(19) Europäisches Patentamt
European Patent Office
Office européen des brevets

(11) EP 2 667 855 B1

(12) **EUROPEAN PATENT SPECIFICATION**

(45) Date of publication and mention of the grant of the patent:
**09.08.2017 Bulletin 2017/32**

(21) Application number: **12701773.9**

(22) Date of filing: **27.01.2012**

(51) Int Cl.:
***A61K 9/16*** (2006.01) ***A61K 31/704*** (2006.01)

(86) International application number:
**PCT/GB2012/050179**

(87) International publication number:
**WO 2012/101455 (02.08.2012 Gazette 2012/31)**

(54) **DRUG DELIVERY SYSTEMS**

WIRKSTOFFFREISETZUNGSSYSTEME

SYSTÈME D'ADMINISTRATION DE MÉDICAMENTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.01.2011 GB 201101429**

(43) Date of publication of application:
**04.12.2013 Bulletin 2013/49**

(73) Proprietor: **Biocompatibles UK Ltd.**
**Farnham, Surrey GU9 8QL (GB)**

(72) Inventors:
- **ASHRAFI, Koorosh**
**Farnham**
**Surrey GU9 8QL (GB)**
- **LEWIS, Andrew Lennard**
**Farnham**
**Surrey GU9 8QL (GB)**
- **HEAYSMAN, Clare**
**Farnham**
**Surrey GU9 8QL (GB)**
- **LLOYD, Andrew**
**East Sussex**
**Sussex BN26 5UY (GB)**
- **PHILIPS, Gary**
**East Sussex**
**Sussex BN26 6TB (GB)**

(74) Representative: **BTG plc Intellectual Property Group**
**5 Fleet Place**
**London EC4M 7RD (GB)**

(56) References cited:
**WO-A1-01/68720 WO-A2-01/72281**
**WO-A2-01/72281 WO-A2-03/053414**

- **ALISA L. BECKER ET AL: "Tuning the Formation and Degradation of Layer-by-Layer Assembled Polymer Hydrogel Microcapsules +", LANGMUIR, vol. 25, no. 24, 15 December 2009 (2009-12-15), pages 14079-14085, XP55021350, ISSN: 0743-7463, DOI: 10.1021/la901687a**
- **Stephen J. Mather: "Current Directions in Radiopharmaceutical Research and DevelopmentCurrent Directions in Radiopharmaceutical Research and Development", 1996, Kluwer Academic Publishers, XP002673735, page 83**
- **ALEXANDER N. ZELIKIN ET AL: "Disulfide-Stabilized Poly(methacrylic acid) Capsules: Formation, Cross-Linking, and Degradation Behavior", CHEMISTRY OF MATERIALS, vol. 20, no. 8, 1 April 2008 (2008-04-01), pages 2655-2661, XP55021354, ISSN: 0897-4756, DOI: 10.1021/cm703403p**
- **YAN YAN ET AL: "Uptake and Intracellular Fate of Disulfide-Bonded Polymer Hydrogel Capsules for Doxorubicin Delivery to Colorectal Cancer Cells", ACS NANO, vol. 4, no. 5, 25 May 2010 (2010-05-25), pages 2928-2936, XP55021356, ISSN: 1936-0851, DOI: 10.1021/nn100173h**
- **Alisa L. Becker ET AL: "Tuning the Formation and Degradation of Layer-by-Layer Assembled Polymer Hydrogel Microcapsules +", Langmuir, vol. 25, no. 24, 15 December 2009 (2009-12-15), pages 14079-14085, XP055021350, ISSN: 0743-7463, DOI: 10.1021/la901687a**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- STEPHEN J MATHER ED - STEPHEN J MATHER: "Current directions in radiopharmaceutical research and development , Hypoxia Agents", 1 January 1996 (1996-01-01), CURRENT DIRECTIONS IN RADIOPHARMACEUTICAL RESEARCH AND DEVELOPMENT; [DEVELOPMENTS IN NUCLEAR MEDICINE ; 30], KLUWER ACADEMIC PUBLISHERS, DORDRECHT [U.A.], PAGE(S) 83, XP002673735, ISBN: 978-0-7923-4254-0

## Description

### Field of the Invention

The present invention relates to novel drug delivery systems which are responsive to hypoxic conditions.

### Background of the Invention

Adequate supplies of oxygen are essential for the normal functioning of all multicellular organisms/metazoan species. The principle energy source for the majority of cellular processes is adenosine triphosphate (ATP). Oxidative phosphorylation is the process by which cells generate ATP from the catabolism of glucose and fatty acids, and the oxygen molecule is the metabolic substrate for this cellular respiration, acting as the terminal electron acceptor.

Low cellular oxygen concentrations, known as hypoxia, can compromise cell function and lead to cell death and tissue damage. However, oxygen molecules are potentially toxic, since they can cause oxidative damage to macromolecules within the cell. The control of oxygen homeostasis is thus essential for maintaining cellular and therefore whole organism viability.

The physiological oxygen tension within healthy human tissue varies according to the organ, but generally lies within the range 20 - 120 mmHg. Cells in different tissues experience different oxygen tensions. Cells within one tissue experience a range of oxygen tensions, depending on both distance from the nearest blood supply (the diffusion distance of oxygen through tissue is estimated to be around 150 μm) and the oxygenation status of that blood supply. For instance, the pressure of $O_2$ of the blood supply to the liver ranges from 95-105 mmHg in the hepatic artery, 50-65 mmHg in the portal vein, 35-45 mmHg in the sinusoids and 30- 40 mmHg in the central vein. The different oxygen tensions that each cell experiences in a healthy liver are all perceived as normal. Indeed, the oxygen gradient along the sinusoid is the regulatory factor which creates the zonation of metabolic functioning within the sinusoid. Sensitive and adaptive mechanisms for sensing intracellular oxygen concentrations and responding to change are vital in order to maintain oxygen homeostasis. Adaptations to fluctuating oxygen levels need to be flexible and capable of responding to subtle changes.

Hypoxia occurs when oxygen tensions are ≤ 7 mmHg, usually as a result of decreased partial pressure of oxygen in the blood (hypoxeamia) or a decrease in the oxygen carrying capacity of the blood (anaemia). Molecular responses to hypoxia result in the increased expression of genes involved in adaptations to hypoxia, and the promotion of a range of physiological responses and adaptations that allow cells to survive in a hypoxic environment. These target genes play critical roles in angiogenesis, metabolism, cell proliferation and cell survival. The master regulator of adaptations to hypoxia is the transcription factor hypoxia-inducible factor (HIF). Fig. 1 is schematic showing that as the oxygen levels supplying the tumour decrease consequently the presence of growth factors increase as the tumour becomes hypoxic and hence increases in size.

Given the adverse metabolic changes that occur within tissues when they experience hypoxia, there is an opportunity to develop smart drug delivery systems that modulate release of active compounds in response to the hypoxic environment that may combat these changes. There has been an increasing interest in stimuli-responsive polymers, particularly in the fields of controlled and self-regulated drug delivery. Delivery systems based on these polymers are developed to closely resemble the normal physiological processes of the diseased state, ensuring optimum drug release according to the physiological need. Polymer architectures have been developed that exhibit a large change in their physico-chemical behaviours in response to minor signals from the environments and have been used in the fabrication of potentially useful materials for pharmaceutical and biomedical applications. The most advanced stimuli-responsive drug delivery systems have also explored a new strategy to design targeted delivery systems to treat complex diseases like cancer and related tumours.

The interconversion of thiols and disulfides is an important biological process with regard to conformational integrity of many proteins. Polymers that contain disulfide functionality can be considered both redox and thiol responsive as they will undergo reversible conversion by both mechanisms (see Meng, F. et al, Reduction-sensitive polymers and bioconjugates for biomedical applications, Biomaterials, 30, (2009), 2180-2198). Glutathione (GSH) is a naturally occurring reducing agent, abundantly present in most cells at a concentration of 10mM, some 5000 times higher than its extracellular levels, which provides a mechanism for triggering a response within the cellular environment. Moreover, hypoxic tumours are known to possess a reductive environment, whereby NAD(P)H dependent cytochrome P450s and other haemoproteins in hypoxic conditions mediate two-electron reduction of a wide range of substrates. This is the basis of some bioreductive drugs, such as those containing low toxicity N-oxides which can be converted to respective tertiary amines that possess antitumour activity, such as banoxantrone (AQ4 → AQ4N conversion under hypoxia, Patterson L.H., Cancer Metastasis Rev. 12, (1993), 119-34).

Oupicky has described poly-L-lysine systems containing disulfide bonds which are reducible in the intracellular environment, providing a mechanism to facilitate DNA release and increasing transfection efficiency by 60-fold (Oupicky, D., Design and development strategies of polymer materials for drug and gene delivery applications, Advanced Drug

Delivery Reviews, 60, (2008) 957).

Microsphere compositions with disulfide cross-links have been described whereby the microspheres are composed of proteins. For instance, Mak *et al* report a non-chemical cross-linking method used to produce pure protein microparticles with an innovative approach, so-called protein activation spontaneous and self-assembly (PASS). This fabrication of protein microparticles is based on the idea of using the internal disulfide bridges within protein molecules as molecular linkers to assemble protein molecules into a microparticle form. The assembly process is triggered by an activating reagent-dithiothreitol (DTT), which is only involved in the intermediate step without being incorporated into the resulting protein microparticles (Mak W.C., et al, Protein particles formed by protein activation and spontaneous self-assembly, Advanced Functional Materials, 20, (2010), 4139-4144). United States Patent Application 20070231400 (Quirk, S.) provides proteinoid microspheres made up of a mixture of thermally condensed amino acids that are cross-linked with a cross-linking reagent. The proteinoid microspheres may be used to encapsulate a material or a compound and to provide slow, sustained or timed release of the material or compound.

Biodegradable polymeric microcapsules based on thiol-disulfide chemistry have been described. Zelikin *et al* prepared poly(methacrylic acid) (PMA) cross-linked with disulfides by layer-by-layer deposition of thiolated PMA (PMASH) and poly(vinylpyrollidone) (PVP) on silica particles, followed by oxidation of the thiols to cross-link the PMA and removal of the silica and PVP by changing the pH to disrupt hydrogen bonding and form a capsular structure. (Zelikin, A.N., et al., Disulfide cross-linked polymer capsules: en route to biodeconstructible systems, Biomacromolecules, 7, (2006), 27-30).

Others have described the development an oral thiomer-based microparticulate delivery systems for insulin by ionic gelation (Greimel A., et al., Oral peptide delivery: in vitro evaluation of thiolated alginate/poly(acrylic acid) microparticles, J. Pharm. Pharmacol., 59, (2007), 1191-8). The microparticulate matrix consisted of either poly(acrylic acid)-cysteine (PAA-Cys) and alginate-cysteine (Alg-Cys) or the corresponding unmodified polymers (PAA, Alg). The mean particle size of all formulations ranged from 400 to 600 microns.

Similarly, a novel mucoadhesive microparticulate drug delivery system has been reported (Bernkop-Schnurch A., et al. Preparation and in vitro characterization of poly(acrylic acid)-cysteine microparticles, J. Controlled Release, 18, (2003), 29-38). Microparticles were prepared by the solvent evaporation emulsion technique using a poly(acrylic acid)-cysteine conjugate of an average molecular mass of 450 kDa with an amount of 308 micromol thiol groups per gram polymer.

Yan *et al.* describe the use of the 3 micron diameter PMASH *microcapsules* described previously [0008] for loading doxorubicin and delivering the drug to colon cancer cells *in vitro* (Yan, Y., et al., Uptake and Intracellular fate of disulfide-bonded polymer hydrogel capsules for doxorubicin delivery to colorectal cancer cells, ACS Nano, 4, (2010), 2928-36). No demonstration of the redox-triggered release of the drug, however, is presented in the paper.

It is disclosed in EP 1007101 A2 that polymer microspheres can be useful in the treatment of embolisation. Embolisation involves the introduction of embolic agents into the arteries feeding a tumour to starve it of its nutrients and oxygen. Drug eluting beads have been developed which in addition to the ischemia induced by the device, administer a concomitant dose of a chemotherapeutic drug, locally to the tumour in a sustained fashion, reducing systemic exposure and maximising drug levels in the tumour (see, for instance, WO 04/071495). There is evidence from pre-clinical tumour models, that embolisation alone may induce hypoxia in the tumour which in turn can initiate a number of pro-survival pathways *via* activation of hypoxia-inducible factor 1$\alpha$ (HIF-1$\alpha$), leading to a more malignant phenotype with increased drug resistance and pro-angiogenic responses (Liang, B., et al., Correlation of hypoxia-inducible factor 1 alpha with angiogenesis in liver tumors after transcatheter arterial embolisation in an animal model, CVIR, 33, (2010) 806-812). Additional prior art includes WO 01/68720 A1 which discloses embolic compositions comprising macromonomers that form hydrogels useful in embolization. The prior art has failed to disclose the use of hydrogel microparticles or microspheres containing disulfide groups for the controlled delivery of drugs in hypoxic environments.

## Summary of the Invention

In accordance with a first aspect of the invention, there are provided microparticles comprising a gel body, wherein the gel body comprises a synthetic polymer and a drug, wherein the microparticles have an average diameter in the range 40 to 1500 $\mu$m, wherein the polymer is cross-linked by groups comprising disulfide linkages and is in the form of a hydrogel.

In accordance with a second aspect of the invention, there are provided microparticles for use in a method of treatment by embolisation, wherein the microparticles comprise a polymer and a drug, wherein the polymer is cross-linked by groups comprising disulfide linkages, and in the treatment drug is released.

In accordance with a third aspect there are provided microparticles having an average diameter in the range 40-1200 $\mu$m comprising a polymer and a drug, wherein the polymer is cross-linked by groups comprising disulfide linkages, and the polymer is a vinyl alcohol polymer.

In accordance with a fourth aspect there are provided microparticles having an average diameter in the range

40-1200 μm comprising a polymer and a cationically charged drug, wherein the polymer is cross-linked by groups comprising disulfide linkages, wherein each cross-linking group comprises 2 anionically charged groups arranged symmetrically about the disulfide unit, wherein said anionically charged groups are electrostatically associated with the drug.

In accordance with a fifth aspect there are provided microparticles having an average diameter in the range 40-1200 μm comprising a polymer and a drug, wherein the polymer is cross-linked by groups comprising disulfide linkages, for use in a method of treatment of a tumour.

The present invention provides a novel drug delivery system useful in the chemoembolotherapy of solid tumours. The system is composed of polymer microparticles containing disulfide cross-links within the structure. The rate of drug release from the microparticles is altered when the tumour becomes hypoxic, leading to an increased release of drug as the disulfide cross-links cleave within the reductive environment of the tumour. The present invention may be used for the local targeting of tumours by either direct intratumoural administration or delivery into the tumour vasculature *via* an intra-arterial route.

<u>Detailed Description of the Invention</u>

The present invention is concerned with a class of responsive polymers that are sensitive to the redox environment or presence of thiols, i.e. are redox/thiol responsive. The polymers are covalently cross-linked with disulfide linkages, although other cross-linkages may also be present. The following schematic shows the thiol and redox response.

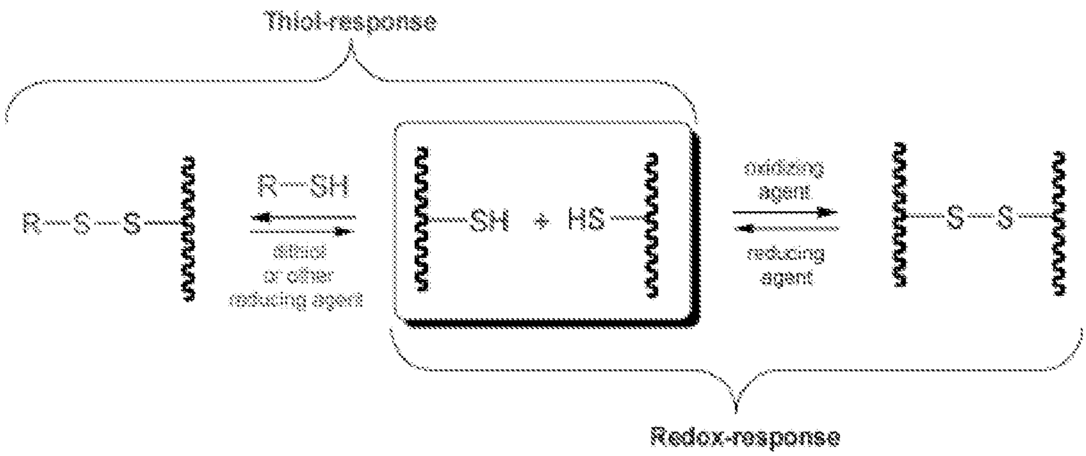


The chemical structures of some of the more common disulfide-containing crosslinkers that may be used in the invention are shown below.

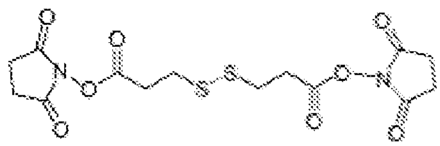
dithiobis(succinimidylpropionate) (DSP)

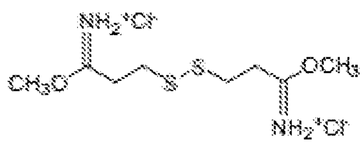
dimethyl-3,3'-dithiobispropionimidate·2HCl

(DTBP)

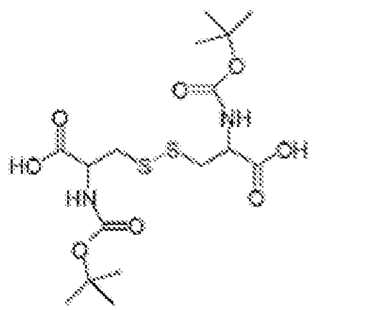

N, N-bis-(tertbutoxycarbonyl)cystine(BC)

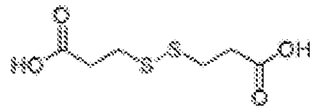

dithiodipropionic acid

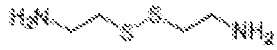

cystamine

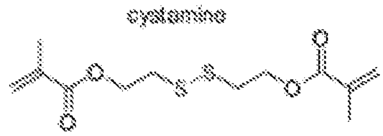

bis(2-methacryloyloxyethyl) disulfide

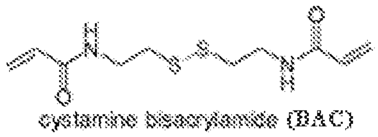

cystamine bisacrylamide (BAC)

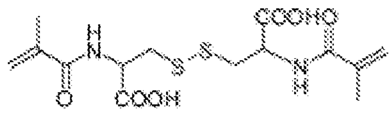

L-cystine bisacrylamide **(BALC)**

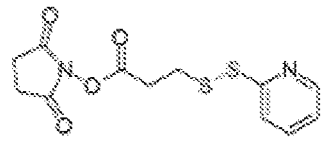

N-Succinimidyl 3-(2-pyridyldithio)propionate (SPDP)

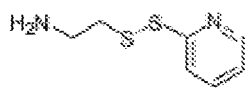

2-(pyridyldithio)-ethylamine (PDA)

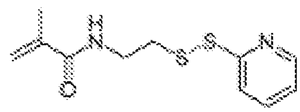

N-[2-(2-pyridyldithio)]ethyl methacrylamide (PDTEMA)

The cross-linker agent typically has general formula:

$$A\text{-}(CH_2)_n\text{-}S\text{-}S\text{-}(CH_2)_{n'}\text{-}A'$$

wherein A and A' are independently selected from groups which can form a covalent bond with the polymer; n and n' are integers ranging from 1 to 10 and are preferably in the range 2-4. n and n' are each most preferably 2.

The $-(CH_2)_n-$ and $-(CH_2)_{n'}$ groups may be substituted with other groups, for instance which may electrostatically associate with the drug. Ionic interactions are preferred. Particularly preferred substituents are carboxyl groups which can form ionic bonds with a cationic drug.

Groups A and A' may be groups susceptible to nucleophilic attack from the polymer. Typically the groups susceptible to nucleophilic attack comprise a group -C(=O)LG wherein LG is a leaving group, and leaves in the reaction with the polymer. Alternatively the group undergoes polymerisation with the polymer. To do this it may comprise an ethylenically unsaturated group, for instance:

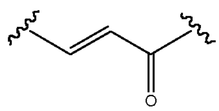

Preferably, the cross-linking agent is symmetrical about the di-sulfide unit. A particularly preferred cross-linking agent has formula:

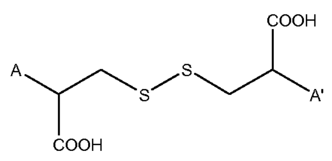

where groups A and A' are as given above.

The cross-linker is typically incorporated into the polymer by reacting the cross-linking agent with pendant ethylenically-unsaturated groups on the polymer.

Polymers useful in this invention include acrylic polymers, vinyl alcohol polymers and acrylate polymers. Preferably the polymer is a copolymer, for instance from the acrylic family such as polyacrylamides and their derivatives, polyacrylates and their derivatives as well as polyallyl and polyvinyl compounds. The polymer may be synthetic, i.e. not natural in origin (such as a protein).

Microparticles may have any shape. The diameter of the microparticles is likely to adopt a so-called "normal distribution", with very few particles having extreme diameters and the majority having average diameters. The microparticles of the invention are preferably microspheres. Microspheres in this invention are spherical, or substantially spherical beads formed from a polymer. Substantially spherical means that a population of microspheres has sphericity measurements centered near about 95%, within a range of about 80% to about 100%. The population may include some individual microspheres which have a lower sphericity measurement, while maintaining the high sphericity measurement for the population as a whole. Sphericity measurements of microspheres can be made on a bulk scale using the Beckman-Coulter(TM) RapidVUE(R) particle analysis system (Hialieah, Fla.).

Generally the microparticles, are formed from a polymeric matrix which is substantially continuous throughout the body of the microparticle, that is, a gel body, ie. there is no interior space as found in the body of a microcapsule.

Preferably, the microparticles are formed from a matrix of water-swellable water-insoluble polymer. Absorbed in the matrix is a therapeutic agent (drug). The polymer preferably has an overall anionic charge at a pH in the range 6 to 8. Typically, the microparticles, when swollen to equilibrium in water, have particle sizes (average diameters) in the range 40-1500 μm, preferably 40-1200 μm, 40-1000 μm, most preferably 40-500 μm or 40-300 μm. Preferably at least 50% of the particles are in the range, more preferably at least 80%, more preferably at least 90%, most preferably at least 95%. The particle size distribution is preferably monomodal.

The average diameter of microparticles may be determined by methods such as fluidized bed separation and sieving, also called screen filtering. Particularly useful is sieving through a series of sieves appropriate for recovering samples containing microparticles of desired sizes. The average diameter can then be determined by techniques such as optical microscopy imaging using a graticule eye piece. In this way the average diameter of a microparticle can be determined, for instance by application of Equivalence Circle diameter. For microspheres which are substantially spherical, the average diameter relates to the actual diameter of the microsphere.

The microparticles are preferably biodegradable. Biodegradation is the chemical breakdown of materials by the actions of living organisms which leads to changes in the physical properties. A microparticle is biodegradable if in the presence of a reducing environment, the disulfide links within the microparticles cleave to their thiol groups and then subsequently cleave, for example to form amino acids which are solubilised and result in the degradation of the microparticle.

Tumours in a state of hypoxia provide a highly reducing environment which can be used as a target for smart drug delivery systems. In a specific example, in the presence of the reducing environment the disulfide links within BALC microparticles cleave to their thiol groups. The reduced BALC is cleaved to its starting component the amino acid L-cysteine, but is held within the microparticle by the copolymer structure, therefore the delivery system retains its permanent embolisation function. However, as the concentration of BALC increases and that of the copolymer decreases within the microparticle, then more BALC will polymerise with itself. When the microparticles are exposed to the biological agents found within hypoxic tumours, at high levels of cross linking the BALC will again cleave to form amino acids, but may be attached to one or more additional L-cysteine molecules in the form of oligomers. The amino acid oligomers may become solubilised once disconnected from the copolymer structure leading to degradation of the microparticle.

The polymer in the invention is preferably water-swellable, but water-insoluble. In the presence of aqueous liquid, therefore, the polymer will form a hydrogel. The polymer is covalently cross-linked, although it may be appropriate for the polymer to be ionically cross-linked, at least in part. The polymer may be formed by polymerising ethylenically unsaturated monomers in the presence of di- or higher-functional cross-linking monomers, the ethylenically unsaturated monomers including an anionic monomer. Copolymers of hydroxyethyl methacrylate, acrylic acid and cross-linking monomer, such as ethylene glycol dimethacrylate or methylene bisacrylamide, as used for etafilcon A based contact lenses may be used. These cross-linking monomers may be present, in addition to the cross-linking agents comprising a disulfide linkage which can participate in polymerisation reactions.

Another type of polymer which may be used to form the water-swellable water-insoluble matrix is polyvinyl alcohol cross-linked using aldehyde type cross-linking agents such as glutaraldehyde. For such products, the polyvinyl alcohol must be rendered anionic, for instance by providing pendant anionic groups by reacting a functional acidic group containing monomer with the hydroxyl groups. Examples of suitable reagents are di-acids, for instance dicarboxylic acids.

The invention is of particular value where the polymer matrix is formed of a polyvinyl alcohol macromer, having more than one ethylenically unsaturated pendant group per molecule, by copolymerisation with ethylenically unsaturated monomers including an acidic monomer. The PVA macromer may be formed, for instance, by providing PVA polymer,

of a suitable molecular weight such as in the range 1000 to 500,000 D, preferably 10,000 to 100,000 D, with pendant vinylic or acrylic groups. Pendant acrylic groups may be provided, for instance, by reacting acrylic or methacrylic acid with PVA to form ester linkages through some of the hydroxyl groups. Methods for attaching vinylic groups capable of polymerisation onto polyvinyl alcohol are described in, for instance, US 4,978,713 and, preferably, US 5,508,317 and 5,583,163. Thus the preferred macromer comprises a backbone of polyvinyl alcohol to which is linked, via a cyclic acetal linkage, to an (alk)acrylaminoalkyl moiety. Example 1 of WO2004/071495 describes the synthesis of an example of such a macromer known by the name nelfilcon B which is useful in this invention. Preferably the PVA macromers have about 2 to 20 pendant ethylenic groups per molecule, for instance 5 to 10.

Where PVA macromers are copolymerised with ethylenically unsaturated monomers including an acidic monomer, preferred acidic monomers are given by general formula I in WO 04/071495.

One particularly preferred type of monomer is an (alk)acrylamido alkanesulfonic acid, such as 2-acrylamido-2-methyl-1-propane-sulfonic acid (AMPS).

There may be included in the ethylenically unsaturated monomer diluent monomer, for instance non-ionic monomer. Such monomer may be useful to control the $pK_a$ of the acid groups, to control the hydrophilicity or hydrophobicity of the product, to provide hydrophobic regions in the polymer, or merely to act as inert diluent. Examples of non-ionic diluent monomer are, for instance, alkyl (alk) acrylates and (alk) acrylamides, especially such compounds having alkyl groups with 1 to 12 carbon atoms, hydroxy, and di-hydroxy-substituted alkyl(alk) acrylates and -(alk) acrylamides, vinyl lactams, styrene and other aromatic monomers.

The ethylenically unsaturated monomer may also include zwitterionic monomer, for instance to increase the hydrophilicity, lubricity, biocompatibility and/or haemocompatibility of the particles. Suitable zwitterionic monomers are described in our earlier publications WO-A-9207885, WO-A-9416748, WO-A-9416749 and WO-A-9520407. Preferably a zwitterionic monomer is 2-(methacryloyloxyethyl phosphorylcholine) (MPC).

In the polymer matrix, the level of anion is preferably in the range 0.1 to 10 meq $g^{-1}$, preferably at least 1.0 meq $g^{-1}$.

Where PVA macromer is copolymerised with other ethylenically unsaturated monomers, the weight ratio of PVA macromer to other monomer is preferably in the range of 50:1 to 1:5, more preferably in the range 20:1 to 1:2. In the ethylenically unsaturated monomer the anionic monomer is preferably present in an amount in the range 10 to 100 mole%, preferably at least 25 mole%.

Preferably the water-insoluble water-swellable polymer has an equilibrium water content measured by gravimetric analysis of 40 to 99 weight %, preferably 75 to 95%.

The polymer may be formed into particles in several ways. For instance, the cross-linked polymer may be made as a bulk material, for instance in the form of a sheet or a block, and subsequently be comminuted to the desired size. Alternatively, the cross-linked polymer may be formed as such in particulate form, for instance by polymerising in droplets of monomer in a dispersed phase in a continuous immiscible carrier. Examples of suitable water-in-oil polymerisations to produce particles having the desired size, when swollen, are known. For instance US 4,224,427 describes processes for forming uniform spherical beads of up to 5 mm in diameter, by dispersing water-soluble monomers into a continuous solvent phase, in a presence of suspending agents. Stabilisers and surfactants may be present to provide control over the size of the dispersed phase particles. After polymerisation, the cross-linked microparticles are recovered by known means, and washed and optionally sterilised. Preferably the particles e.g. microparticles, are swollen in an aqueous liquid, and classified according to their size.

The cross-linking agent comprising a disulfide linkage is incorporated into the polymer as illustrated in the Examples. The agent is typically incorporated into the aqueous phase when reverse suspension polymerisation is used. If the cross-linking agent comprises ethylenically unsaturated groups it can participate in a polymerisation reaction with the polymer. Preferably, the microparticle formulation contains 0.5-100% by wt, more typically 0.5-80% by weight of the crosslinker.

Any drug which can be incorporated into the microparticles of the invention may find utility in this invention. The drug may be, for instance, selected from the group consisting of anti-tumour drugs, anti-angiogenesis drugs, anti-fungal drugs, antiviral drugs, anti-inflammatory drugs, anti-bacterial drugs, anti-histamine drugs, antineoplastic drugs, enzymes and anti-allergenic agents. Preferred drugs are anti-neoplastic drugs and have chemotherapeutic properties. These may be used in anti-tumour therapy.

The drug may be a cytotoxic agent. A cytotoxic agent is one that is directly toxic to cells, preventing their reproduction or growth. Suitable cytotoxic agents are, for instance, anthracycline compounds such as doxorubicin and other compounds disclosed in WO04071495, camptothecin derivatives as described in WO2006027567, taxanes, platinum-based neoplastic antimetabolites such as 5-FU, FUDR, mercaptopurine, capecitabine, other cytotoxic antibiotics such as actinomycin D and vinca alkaloids, including vinblastine, vincristine, vindesine and vinorelbine. Examples also include cytarabine, gemcitabine, cyclophosphamide, fludaribine, clorambucil, busulfan, mitoxantrone, retinoids, anagrelide etc. Camptothecin compounds such as topotecan and irinotecan are particularly preferred as they are charged.

Other useful drugs include the platins (cisplatin, oxaliplatin, carboplatin), the taxanes (paclitaxel, docetaxel) and bleomycin.

One class of cytotoxic or cytostatic compounds which may be used comprises rapamycin and rapamycin analogues, which target mTOR. Such compounds include sirolimus, temsirolimus, everolimus, tacrolimus, pimecrolimus, zotarolimus, biolimus, and AP23573. Any of the compounds encompassed within the scope of rapamycin analogues described in WO-A-2003022807, the contents of which are incorporated herein by reference, may be used as the rapamycin analogue.

Other suitable drugs include multikinase inhibitors such as, but not limited to, sorafenib, sunitinib, bosutinib, brivanib, axitinib, bortezomib, imantinib, canertinib, dasatinib, dovitinib, gefitinib, lapatinib, lestaurtinib, masitinib, mubritinib, nilotinib, pazopanib, saracatinib, tacocitinib, tozasertib, vandetanib and vatalanib.

Other suitable drugs include etinostat, enzastaurin and PARP inhibitors such as olaparib.

The drug may alternatively be a COX-inhibitor. COX-inhibitors, for instance NSAIDs, could act as anti-inflammatory and analgesic agents, targeting inflammation and pain associated with the chemoembolisation procedure. One example is Ibuprofen.

The therapeutic active (drug) used in the present invention is preferably an anthracycline compound, which comprises an anthraquinone group to which is attached an amine sugar. The amino group on the sugar is believed to associate with any anionic groups in the polymer matrix, to allow high levels of loading and controlled delivery after administration.

Preferred drugs are the anthracyclines, for instance doxorubicin, epirubicin, daunorubicin, idarubicin and the anthracenedione mitoxantrone.

In the invention the drug is generally not covalently attached to the polymer matrix.

The drug may be ionic or non-ionic. When the drug is cationic, it is preferred that the cross-linker has anionic groups which may help bind the drug.

Preferably there are two anionic groups and these are distributed symmetrically about the disulfide linkage. The cross-linking agent L-cystine bisacrylamide is particularly preferred in this regard. The two groups bind cationic drug molecule. Without being bound by theory, it is believed that when the disulfide linkage is cleaved in a hypoxic environment the two anionic groups are forced apart, which facilitates release of the drug.

Where the polymer is cationic, the drug is preferably anionic, and electrostatically associated with the polymer.

It is also possible for the polymer, or the drug not to be charged.

The therapeutic active (drug) may be incorporated into the polymer matrix by a variety of techniques. In one method, the therapeutic active may be mixed with a precursor of the polymer, for instance a monomer or macromer mixture or a cross-linkable polymer and cross-linker mixture, prior to polymerising or cross-linking. Alternatively, the active may be loaded into the polymer after it has been cross-linked. For instance, particulate dried polymer may be swollen in a solution of therapeutic active, preferably in water, optionally with subsequent removal of non-absorbed agent and/or evaporation of solvent. A solution of the active, in an organic solvent such as an alcohol, or, more preferably, in water, may be sprayed onto a moving bed of particles, whereby drug is absorbed into the body of the particles with simultaneous solvent removal. Most conveniently, we have found that it is possible merely to contact swollen particles suspended in a continuous liquid vehicle, such as water, with a solution of drug, over an extended period, whereby drug becomes absorbed into the body of the particles. This is believed to be analogous to a cation exchange type process. The swelling vehicle may subsequently be removed or, conveniently, may be retained with the particles as part of the product for subsequent use as an embolic agent.

The microparticles are then separated from the swelling vehicle, filtered and dried. Details of suitable processes are given in WO 04/071495.

The composition which is administered to a patient in need of embolotherapy having a solid tumour, for instance a hepatocellular carcinoma, is an aqueous suspension of swollen particles containing absorbed drug. It is often desirable for the suspension to be mixed prior to delivery with an imaging agent such as a conventional radiopaque agent, as is used for gel type embolic compositions. For example, an aqueous suspension of swollen particles containing absorbed drug may be mixed immediately prior to administration with a liquid radiopaque agent conventionally used with embolic agents, e.g. lipiodol, in amounts in the range 2:1 to 1:2, preferably about 1:1 by volume.

Further details of this are given in WO 04/071495.

Brief description of drawings

Figure 1 is a schematic showing that as the oxygen levels supplying the tumour decrease consequently the presence of growth factors increase as the tumour becomes hypoxic and hence increases in size

Figure 2A shows the size distribution of PVA macromer only microspheres

Figure 2B shows the size distribution of PVA macromer microspheres with 1.7% BAC

Figure 3 shows the size distribution of PVA macromer microspheres with 4% BAC

Figure 4 shows the size distribution of PVA macromer microspheres with 8% BAC
Figure 5 shows the size distribution of PVA macromer microspheres with 2% BALC
Figure 6 shows the size distribution of PVA macromer microspheres with 4% BALC
Figure 7 shows the size distribution of PVA macromer microspheres with 8% BALC
Figure 8 shows the size distribution of PVA macromer microspheres with 15% BALC
Figure 9 shows the percentage of cross-linked BALC microspheres v doxorubicin loading
Figure 10 shows a picture of loaded microspheres after washing of doxorubicin.
Figure 11 shows a scan of DTT effect on Doxorubicin over time
Figure 12A shows an elution profile of doxorubicin from 31% BALC microspheres.
Figure 12B shows an elution profile percentage release: Reduced BALC Vs Non-Reduced BALC
Figure 13 shows the percentage difference reduced and non-reduced BALC microspheres
Figure 14A shows a light microscope image of 100% BALC microparticles:

Figure 14B shows a picture of 100% BALC microparticles settling in water

Figure 15 shows the size of the 45% BALC microspheres before and after reduction
Figure 16 shows an elution profile of Irinotecan from 31% BALC microspheres (N=3)
Figure 17 shows rapamycin loaded BALC microspheres
Figure 18 shows diclofenac loaded BALC microspheres
Figure 19 shows BALC-HEMA microparticle elution of doxorubicin
Figure 20A shows BALC-HEMA microparticles loaded with drug prior to elution
Figure 20B shows BALC-HEMA microparticles during elution in a normal environment
Figure 20C shows BALC-HEMA microparticles during elution in a reducing environment
Figure 21 shows BALC-AA microparticle elution of doxorubicin
Figure 22A shows BALC-acrylic acid microparticles loaded with drug prior to elution
Figure 22B shows BALC-acrylic acid microparticles during elution in a normal environment
Figure 22C shows BALC-acrylic acid microparticles during elution in a reducing environment

Examples

**Example 1: Outline method for the synthesis of disulfide cross-linker *N,N'*-bis acryloyl-(L)-cystine (BALC)**

The disulfide cross-linker named BALC (chemical formula: $C_{12}H_{16}O_6N_2S_2$) was prepared using a modified example presented in the paper "New poly(amido amine)s containing disulfide linkages in their main chain." (2005). Journal of polymer science Part A: polymer chemistry. Vol 43, Issue 7. Pages 1404-1416.

The procedure included amidation and di-acrylation of cystine dihydrochloride (scheme 1).

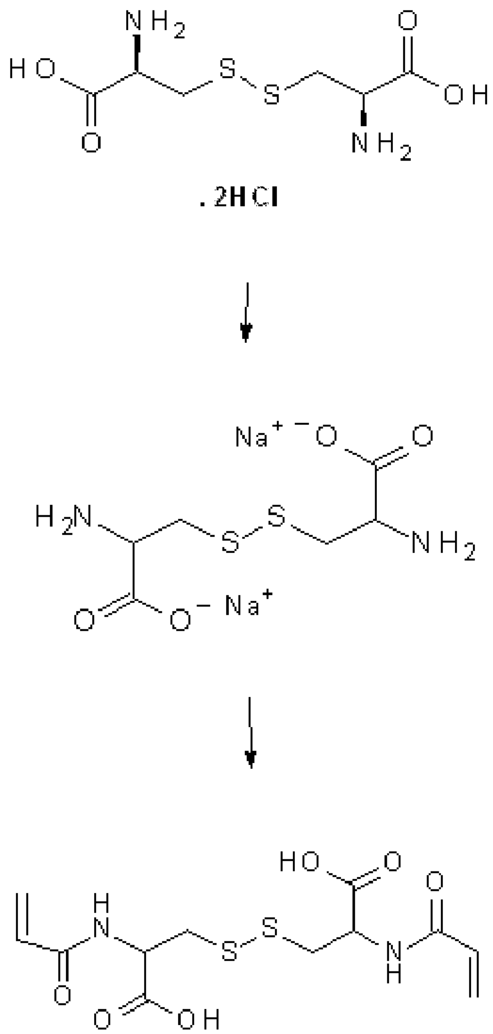

Scheme 1: Synthesis of Bis-acryloyl-L-cystine

In a 3 necked 500 mL flask, which was equipped with an overhead stirrer through the middle outlet, 12.1 g of cystine dihydrochloride and 15 mg 4-hydroxy-2,2,6,6-tetramethylpiperidin-1-oxyl free radicals, (TEMPO) was dissolved in 50 mL of water. The flask was placed in a water bath and the mixture was cooled to 0-5°C with the use of ice and the temperature was observed with a glass thermometer. The over head stirrer was set to 150 rpm and the temperature was maintained at 0-5°C throughout the addition of the materials.

Two glass dropping funnels were placed either side of the stirrer. One of the dropping funnels contained a combination 10 mL of dichloromethane and 10 M acryloyl chloride. The other dropping funnel contained 6 M NaOH. Firstly, 6 M NaOH was added drop-wise until the pH fell to 8-10 and that pH was maintained all the way through the addition which accumulated to approximately 50 mL of NaOH. Once at the correct pH, the acryloyl chloride solution was added manually, drop wise, over a period of 1 hour 30 min. Once the addition had finished, the mixture was allowed to stir at room temp for a further 3 hours. Once stirring was complete, the pH was adjusted to 1-2.

The mixture was frozen over night and then freeze dried. Once freeze drying had finished, acetone was added to the white powder and any NaCl was filtered off. All the acetone was removed and the mixture was dried and then methanol was added. Once the methanol was added the solution was put under rotary evaporation and finally in this method a large quantity of white product was obtained.

In this process 10.15 g of cross-linker was obtained and gave a yield of 58%.

Analysis of the structure BALC was performed using $^1$H NMR which showed proton peaks at 6.3 ppm for H2, 6.24 ppm for H3, 5.8 ppm for H1, 4.7 ppm for H5, 3.3 ppm for H6 and 3.0 ppm for H7.

Analysis of the structure BALC was performed using $^{13}$C NMR which showed carbon peaks at 175 ppm for C6, 168 ppm for C3, 129 ppm for C2, 128 ppm for C1, 53 ppm for C4 and 39 ppm for C5.

Analysis of the structure BALC was performed using Electrospray Mass spectroscopy which confirmed the

molecular weight of 348.

**Example 2: Synthesis of microspheres cross-linked with *N,N'*-bis(acryloyl)cystamine (BAC)**

Microspheres of the invention were made by reverse suspension polymerisation in a 2 litre jacketed vessel with an over head stirrer.

The organic phase was composed of 600 g of n-butyl acetate and 11.5 g of cellulose acetate butyrate. The vessel was purged with nitrogen and a stirrer speed of approximately 400 rpm was utilised.

The aqueous phase consisted of a Nelfilcon B Macromer- a polymerisable macromer from the widely used water soluble polymer PVA, which is modified with N-acryloylaminoacetaldehyde. It is this functionalised PVA macromer that is heavily cross-linked with a disulfide cross-linker producing a microsphere containing reducible disulfide cross-links. The water content of the aqueous phase is made up to 130 g.

The disulfide cross-linking agent used in this example was purchased from Sigma Aldrich Inc. The amount of cross-linker to be used was predetermined by calculating a percentage based on the dry weight content of the aqueous phase. A series of 1.7%, 4% and 8% cross-linked microspheres were produced.

In the case of Bis(acryloyl) cystamine, due to its hydrophobic nature, the cross-linker was dissolved in the amount of water to be added to make up the aqueous phase. The cross-linker was dissolved by heating, however ensuring not to exceed the melting point of the cross-linker. The water was then allowed to cool and the solution was dissolved in the macromer.

The incorporation of the cross-linker into the PVA macromer was carried out with the use of a thermal initiator. After 2 hours the synthesis of the stimuli responsive beads was complete and was removed from the vessel *via* a cleaning process using ethyl acetate and acetone. The size of the microspheres could be calibrated to a desired size by alteration of the stirrer speed.

After removal from the vessel, the beads were washed and cleaned to remove any residual of acetone. After hydrating, the microspheres were dried using an oven and crushed down. Elemental analysis was used to confirm the incorporation of the cross-linker based on the sulfur content.

Elemental analysis of PVA Macromer only microspheres (no cross-linker)

| ELEMENT | C | H | N | S |
|---|---|---|---|---|
| **% Theory** | 53.64 | 9.04 | 1.53 | 0 |
| **% Found 1** | 52.17 | 9.04 | 0.30 | <0.10 |
| **% Found 2** | 52.36 | 9.03 | 0.29 | <0.10 |

1.7% BAC microspheres

| ELEMENT | C | H | N | S |
|---|---|---|---|---|
| **% Theory** | 53.74 | 9.03 | 1.69 | 0.42 |
| **% Found 1** | 53.22 | 9.10 | 0.51 | 0.85 |
| **% Found 2** | 53.15 | 9.15 | 0.51 | 0.92 |

4% BAC microspheres

| ELEMENT | C | H | N | S |
|---|---|---|---|---|
| **% Theory** | 53.87 | 9.02 | 1.92 | 0.99 |
| **% Found 1** | 53.24 | 8.84 | 0.83 | 1.35 |
| **% Found 2** | 53.13 | 8.93 | 0.84 | 1.42 |

8% BAC microspheres

| ELEMENT | C | H | N | S |
|---|---|---|---|---|
| % Theory | 54.10 | 8.99 | 2.3 | 1.98 |
| % Found 1 | 52.54 | 8.86 | 1.37 | 2.32 |
| % Found 2 | 52.62 | 8.99 | 1.42 | 2.33 |

**Example 3: Synthesis of microspheres cross-linked with N,N'-bis(acryloyl)-(L)-cystine**

Microspheres of the invention were made with reverse suspension polymerisation, in almost exactly the same manner as for Example 2.

In this example the BALC was synthesised in-house. The cross-linker was hydrophilic and very soluble in water, unlike BAC; therefore, in the synthesis of BALC microspheres, no heating was required to dissolve the cross-linker in water. Due to the hydrophilic nature of the cross-linker, the percentage incorporation in the microspheres could be increased dramatically from 0% to essentially 100% cross-linked BALC microspheres.

After manufacture of the microspheres of Example 3, they were washed and cleaned as described in Example 2. Again, to confirm incorporation of the disulfide cross-linker in the microsphere, elemental analysis was performed on the different formulations of BALC microspheres. However, instead of drying down the beads in an oven and crushing them, which was performed with the BAC microspheres, a freeze dryer was employed instead.

Elemental analysis of PVA macromer only microspheres- no cross-linker

| ELEMENT | C | H | N | S |
|---|---|---|---|---|
| % Theory | 53.6 | 9.04 | 1.53 | 0 |
| % Found 1 | 52.52 | 9.26 | 0.60 | <0.10 |
| % Found 2 | 52.83 | 9.42 | 0.61 | <0.10 |

4 % BALC microspheres

| ELEMENT | C | H | N | S |
|---|---|---|---|---|
| % Theory | 53.1 | 8.8 | 1.7 | 0.9 |
| % Found 1 | 51.99 | 8.48 | 1.16 | 1.36 |
| % Found 2 | 51.90 | 8.68 | 1.14 | 1.29 |

15 % BALC microspheres

| ELEMENT | C | H | N | S |
|---|---|---|---|---|
| % Theory | 51.8 | 8.4 | 2.4 | 2.7 |
| % Found 1 | 50.67 | 8.38 | 2.04 | 2.82 |
| % Found 2 | 50.50 | 8.33 | 2.17 | 2.74 |

31 % BALC microspheres

| ELEMENT | C | H | N | S |
|---|---|---|---|---|
| % Theory | 49.8 | 7.8 | 3.5 | 5.6 |
| % Found 1 | 48.66 | 7.81 | 3.14 | 5.31 |
| % Found 2 | 48.36 | 7.73 | 3.13 | 5.35 |

**Example 4: Reduction and sizing of BAC microspheres.**

After synthesis of the BAC microspheres, further characterisation studies were performed, focusing on the size of the microspheres and their potential to expand in a reductive environment.

Due to the incorporation of disulfide cross-links throughout the structure of the microsphere, in a reductive environment the disulfide links should cleave leading to an increase in the diameter of the microsphere. This increase in diameter should in theory lead to a much more efficient embolisation of the target vessel.

The new microspheres should act as a permanent embolic and will not degrade on reduction of the disulfide cross-linker. The microspheres are held in place by the PVA backbone.

The microspheres were reduced using a modified example presented in the paper "Redox-Cleavable star cationic PDMAEMA by arm-first approach of ATRP as a nonviral vector for gene delivery," (2010) Biomaterials, Volume 3, Pages 559-569

Of each microsphere formulation 1 mL of microspheres was placed in 10 mL vial with 1 mL of distilled water.

The microspheres were then transferred onto a glass dish for manual sizing under an optical microscope.

The same microspheres were then transferred back into the glass vial and had 1.5 mL of 4% $NaBH_4$ added to the beads to cleave the disulfide bonds.

The beads were then left in a water bath at 37°C for 1 hour.

After 1 hour had elapsed the vials were removed from the water bath and a constant procedure of washing with distilled water was carried out until all the $NaBH_4$ had been removed.

Once all the reducing agent had been washed out the microspheres were placed back in glass dishes and re-analysed under the optical microscope using the same method as before.

Once all the microspheres were sized the data was collated and separated into two groups of the beads before reduction and after reduction (Figures 2A-4). The PVA macromer only microspheres were used as the control group where no size change was expected.

**Example 5: Reduction and sizing of BALC microspheres**

The reduction and sizing of the BALC microspheres were performed using the exact same method of that used for the BAC microspheres. The results are shown in Figures 5-8:

**Example 6: Maximum drug loading capacity of BALC microspheres with doxorubicin**

Due to the use of the disulfide cross-linker BALC, there are two carboxylic acid groups attached to the monomer. At neutral pH the carboxylic groups are deprotonated leaving two negatively charged carboxylate groups which bind positively charged drugs.

Experiments were performed to confirm the BALC microspheres drug binding capabilities and maximum binding capacity.

Theoretical binding capacity was assumed based on a 2:1 ratio of drug to monomer due to the two carboxylate groups.

1 mL of microspheres of each formulation of BALC microspheres would be placed in respective 10 mL glass vials. Also 1 mL of PVA only microspheres was also placed in a glass vial to be used as a control.

Based on the theoretical binding capacity, an excess of Doxorubicin hydrochloride was added to each vial with a constant value of 60 $mg.mL^{-1}$ per vial.

The microspheres were then left to load overnight, under constant agitation on a shaker.

The shaker was then stopped and all of the remaining drug in the vials was removed using a glass pipette and placed in a separate vial for analysis. The microspheres then had 10 mL of distilled water added and left to shake. At a later time point the 10 mL of drug tainted water was then removed and placed in a vial, for analysis, and 10 mL of fresh distilled water was again added to the microspheres.

This process, known as doxorubicin washing, was carried out numerous times until it was clear that no more

drug was being released by the microspheres and that the water was clear of the obvious red colour of the doxorubicin drug.

The collected drug samples from the doxorubicin washing were then analysed using UV-Vis spectroscopy to determine the exact amount of drug removed from the vials, which in turn reveals the binding capacity of the BALC microspheres.

Standard solution of known doxorubicin concentration were used to confirm the exact amount of doxorubicin added to the vials, and the same standards were used to determine the exact amount of drug removed from the vials. The value of the latter was taken away from the initial amount of drug added to determine the amount of bound drug to the BALC microspheres (Figure 9).

Table 1: Loaded amounts of doxorubicin

| BALC microspheres % cross-linked | Theoretical mg.mL$^{-1}$ | Actual mg.mL$^{-1}$ |
|---|---|---|
| 0 | 0 | 2.1 |
| 2 | 2.5 | 5.44 |
| 4 | 5.2 | 8.57 |
| 8 | 10.4 | 11.25 |
| 15 | 19.5 | 14.69 |

The control microspheres did not actively uptake any drug; however the doxorubicin does stain the beads and glassware. (see Figure 10).

After analysis of the loading results to further increase the amount of drug loaded an n=4 loading experiment was performed using a formulation with higher BALC content (31% BALC microspheres).

Again the same method was used to determine the loading capacity of the other BALC microspheres formulations was used to determine the loading capacity of the 31% BALC microspheres.

Table 2: Doxorubicin loaded BALC microspheres

| 31% BALC replicate | Loaded Doxorubicin (mg.mL$^{-1}$) |
|---|---|
| 1 | 34.2 |
| 2 | 39.7 |
| 3 | 41.6 |
| 4 | 37.8 |

Average maximum loading capacity of 31% BALC microspheres = 38.3 mg.mL$^{-1}$

**Example 7: Drug elution from BALC microspheres**

After loading of the BALC microspheres according to Example 6, the release kinetics of drug from the beads was determined.

The null hypothesis set out before testing was that in a hypoxic (reducing) environment, the disulfide would undergo rapid cleavage, at a time scale of minutes to hours, with the quick response chemical degradation leading to a significantly enhanced release of drug from the microspheres compared to those in a non-reducing environment.

The *in vitro* experiment was set up to replicate the possible action of drug loaded BALC microspheres in an *in vivo* environment of hypoxia.

Firstly, six 10 mL glass vials were filled with 1 mL of unloaded 31% BALC microspheres. These microspheres were then loaded to their maximum loading capacity, with an average of 38.3 mg.mL$^{-1}$, and were loaded using the method in Example 6. Once all the doxorubicin was washed away, the microspheres were kept in their separate vials in distilled water until the commencement of the elution.

Then six 220 mL brown jars were taken and filled with 200 mL of phosphate buffered saline solution (PBSS). These jars were placed on a magnetic stirrer plate with a magnetic stirrer in each jar.

Diothiothreitol (DTT) is a well known mild disulfide reducing agent and is used to maintain disulfide groups in their reduced thiol state. This was the chosen reducing agent as UV/Vis spectroscopy scans showed that DTT does not alter the structure of doxorubicin over time. A high concentration of DTT was added to doxorubicin and was scanned at 1 hour time points using a UV/Vis spectrophotometer. After 17 hours of exposure to DTT there was very little change to

the absorbance of the doxorubicin from start to finish (Figure 11).

To produce a reducing environment for the BALC microspheres, DTT was added to three of the brown jars at 50-60mM. The DTT was calculated to be in 10 molar excess to the disulfide cross-linker. The remaining three brown jars were left as PBS and acted as the control showing the elution of the BALC microspheres in a non- reducing environment.

At time 0 hour, 5 mL of the elution media was used to remove the BALC microspheres from the vials and added to the brown jars. At this point the elution started.

At certain time points during release 5 mL of the elution media containing doxorubicin from the BALC microspheres was sampled for analysis by UV-Vis spectroscopy. Immediately after removal of the elution media from the jar, 5 mL of fresh elution media of PBS was added to the three brown jars containing the non-reducing environment. Also 5 mL of PBS with DTT would be added to the three brown jars containing the reducing environment. This helped to maintain the reducing environment and keep the thiols in the reduced state.

The exact amount of drug released from the beads in the two environments was calculated using standard solutions of known doxorubicin concentration. The results are shown in Figures 12A & B:

After the n=3 elution using DTT a model-independent approach using a similarity factor was used to determine if the elutions were significantly different.

The equations to determine whether the elution is significantly different or not is found in the document *Guidance for Industry Dissolution Testing of Immediate Release Solid Oral Dosage Forms.* Among several methods investigated for dissolution profile comparison, $f_2$ is the simplest. Moore and Flanner proposed a model independent mathematical approach to compare the dissolution profile using two factors, $f_1$ and $f_2$ (1).

$$f_1 = \{[\Sigma_{t=1}n|R_t - T_t|]/[\Sigma_{t=1}n\ R_t]\}.100$$

$$f_2 = 50.\log\{[1+(1/n)\Sigma_{t=1}n\ (R_t - T_t)^2]^{-0.5}.100\}$$

where $R_t$ and $T_t$ are the cumulative percentage dissolved at each of the selected n time points of the reference and test product respectively. The factor $f_1$ is proportional to the average difference between the two profiles, where as factor $f_2$ is inversely proportional to the average squared difference between the two profiles, with emphasis on the larger difference among all the time-points. The factor $f_2$ measures the closeness between the two profiles. Because of the nature of measurement, $f_1$ was described as difference factor, and $f_2$ as similarity factor (2). In dissolution profile comparisons, especially to assure similarity in product performance, regulatory interest is in knowing how similar the two curves are, and to have a measure which is more sensitive to large differences at any particular time point. For this reason, the $f_2$ comparison has been the focus in Agency guidances.

The guidelines state that for curves to be considered similar, $f_1$ values should be close to 0, and $f_2$ values should be close to 100. Generally, $f_1$ values up to 15 (0-15) and $f_2$ values greater than 50 (50-100) ensure sameness or equivalence of the two curves and, thus, of the performance of the test (postchange) and reference (prechange) products.

If the values for $f_1$ are not below 15 and values for $f_2$ are not above 50 then it can be taken that the elution is significantly different

Table 3: Similarity data for elution

| | | Comparison of total eluted mean | Comparison of percentage eluted mean |
|---|---|---|---|
| > 50 | $F_2$ | 94.2 | 81.0 |
| | | | |
| < 15 | $F_1$ | 37.21 | 36.69 |

The dissolution test shows that although $f_2$ is greater than 50, $f_1$ is not less than 15 and therefore the two elutions are significantly different.

The same drug elution experiment was performed using 2-mercaptoethanol as the reducing agent instead of DTT, and was carried out on each BALC microsphere formulation. The results again showed that there was an elution difference between the BALC microspheres that were reduced and those that were not reduced. A time point of 360 minutes was chosen to compare the percentage difference between the elutions within each BALC formulation to see

the percentage eluted from each formulation compared against each other (Figure 13).

The results again show a considerable percentage difference between reduced and unreduced elution, which corroborates the previous results. The graph also indicates a possible trend in the release kinetics for the loading of more doxorubicin.

The microspheres may be separated from the swelling vehicle, filtered and dried. Details of suitable processes are given in WO 04/071495.

The composition which is administered to a patient in need of embolotherapy having a solid tumour, for instance a hepatocellular carcinoma, is an aqueous suspension of swollen particles containing absorbed drug. It is often desirable for the suspension to be mixed prior to delivery with an imaging agent such as a conventional radiopaque agent, as is used for gel type embolic compositions. For example, an aqueous suspension of swollen particles containing absorbed drug may be mixed immediately prior to administration with a liquid radiopaque agent conventionally used with embolic agents, e.g. Lipiodol, in amounts in the range 2:1 to 1:2, preferably about 1:1 by volume.

Further details of this are given in WO 04/071495.

**Example 8: Syntheses of BALC microparticles with high percentages of cross linking**

BALC is highly reactive due to its acrylate groups, and although soluble in water, as the concentration of BALC increases, its addition to water can be compromised if added too quickly. It has been observed that BALC may gel when large quantities are added to water. It is assumed that the BALC has polymerised at this point and is no longer soluble in water. Therefore the BALC must be added in very small quantities to the pre-determined volume of water. Only once has the BALC fully dissolved can further BALC be added to the solution. Once fully dissolved, the BALC is dispersed in the PVA macromer and placed on a roller mixer for 10 minutes. To incorporate the BALC successfully, it is necessary to lower the quantity of the thermal initiator added to the reaction as stated in previous lower BALC formulations. By lowering the amount of thermal initiator in the synthesis step to a tenth of its original value, it is possible to successfully increase the amount of BALC within the microspheres.

Using the existing technique, and an increased reaction time due to the lower amount of initiator, BALC beads from 0.5%-100% have been produced. Due to the insolubility of BALC in the organic phase it is possible to produce these microspheres with minimal loss of product as the cross linker does not partition into the organic phase as it polymerises. Nearly all BALC being introduced into the system is incorporated into the beads. The elemental analysis below is within $\pm 10\%$ of the theoretical formulation, which is typical for polymer systems and illustrates the successful synthesis of a 45% BALC microsphere.

Table 4: Elemental analysis of 45% BALC microspheres

| ELEMENT | C | H | N | S |
|---|---|---|---|---|
| % Theory | 48.4 | 7.2 | 3.7 | 8.1 |
| % Found 1 | 45.83 | 7.30 | 3.62 | 7.96 |
| % Found 2 | 45.65 | 7.19 | 3.67 | 7.97 |

A further example was carried out removing all PVA macromer from the reaction mixture, yielding a microsphere bound together purely by BALC. Figure 14A shows a light microscope image of 100% BALC microparticles. Figure 14B shows 100% BALC beads settling in water. These microparticles were harder to compress in comparison to earlier formulations containing PVA but the formulation, more friable but still able to load charged drug *via* ion exchange.

**Example 9: Reduction and Sizing of 45% BALC microspheres**

Although the elemental analysis in example 8 indicated that BALC had been successfully been incorporated into the bead structure, further evidence was sought to support this supposition and also to determine whether the cross linker had remained intact within the bead structure. This was performed using a visual method with the use of 5,5'-dithiobis(2-nitrobenzoic acid).

DTNB (Ellman's reagent) is a chemical that can be used to quantify the amount of thiol groups within a sample. In this case was being used to qualify the presence of the disulfide groups within the microparticle structure. When the microparticles are in a non-reduced state the disulfide groups should not react with the Ellman's reagent, but when the microparticles are reduced, the thiol groups will cleave the disulfide bond within the DTNB to give 2-nitro-5-thiobenzoate ($NTB^-$), which ionizes to the $NTB^{2-}$ dianion in water at neutral and alkaline pH. The mechanism for the cleavage of DTNB to it $NTB^{2-}$ dianion by the thiol group presented on the microparticles can be seen below (scheme 2).

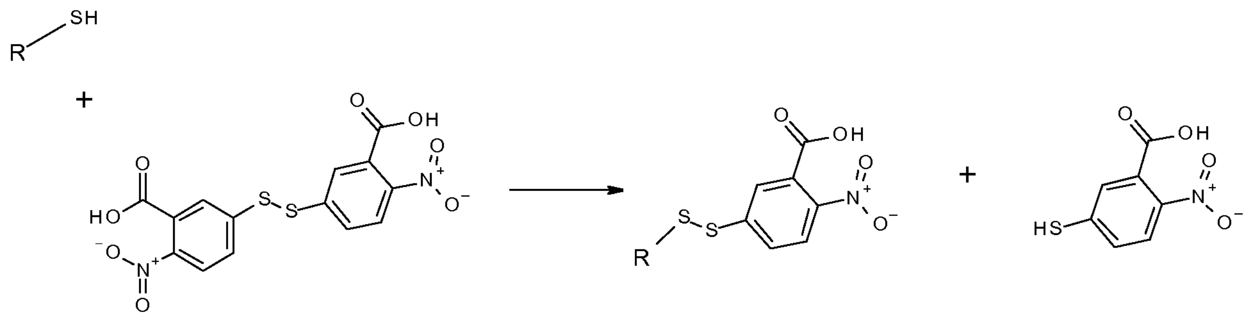

The $NTB^{2-}$ produces a yellow colour which can be used to confirm the incorporation of the BALC. Therefore DTNB was added to microparticles which were not reduced to see if a colour change was observed. As no colour change was noted, this would imply that either the BALC is in its disulfide form or that no BALC had been incorporated. Subsequently, another batch of the same bead formulation was taken and reduced with $NaBH_4$. After reduction, the reducing agent was thoroughly washed away, ensuring none was left in the microparticles, as the reducing agent could yield a false result by reducing the DTNB directly. Once removed the DTNB was added to the reduced microparticles. An immediate colour change to yellow was observed for all formulations. The change in colour only after reduction demonstrates that the BALC has been incorporated into the microparticle in its disulfide form. This shows that the microparticles have the potential to respond to the environment of a hypoxic tumour. This response to a reducing environment in earlier formulations is seen as an increase in diameter. The higher cross linked formulations were tested to determine if they had a similar alteration in their physical properties.

1 mL of 45% BALC microspheres measured under an optical microscope. The microspheres were then placed in a vial with 1 mL 4% $NaBH_4$. The sample was incubated in a water bath for 1 hour at 37°C. The reducing agent was removed as previously described and the beads resized.

The results seen in Fig. 15 demonstrate that the higher BALC microsphere formulations act in exactly the same manner as the previous lower cross linked formulations. The results of the sizing indicate that the beads could respond to a hypoxic environment. All previous lower formulations had an average increase of 200 μm, but the 45% BALC microspheres have an average increase of 300 μm. This represents a potential trend that as the amount of BALC within a formulation increases so does the increase in the diameter of microspheres as more cross links are broken and the swellability of the microparticles increases.

**Example 10: Biodegradability of BALC microparticles**

BALC microparticles were synthesised with the intention of using them as an embolic, occluding the target vessel and starving the tumour of its nutrient supply. It is thought that as the number of the disulfide cross links increase within a formulation, in conjunction with a decrease in PVA macromer content, then at some point the formulation may biodegrade/resorb, as the polymer network is broken into smaller fragments upon reduction of the cross links. Analyses of BALC formulations from 0 - 45% were performed by placing 1 mL in a 200 mL PBS with a reducing agent induced by DTT, and the beads were constantly agitated with the use of a magnetic stirrer. The experiment ran for greater than 72 hours and at that point the structural integrity of the microspheres was examined visually. The majority of beads in each formulation appeared intact at this time point except for a small number of microspheres within the 45% BALC formulation, which appeared to have broken apart. A 100% BALC microparticle may be completely biodegradable. As BALC is the only component of this microparticle, when reduced it could cleave to its amino acid cysteine. At such low molecular weights the bead should disintegrate and absorbed by the body.

**Example 11: Loading and elution of Irinotecan Hydrochloride**

1 mL of 31% BALC microspheres were loaded with 38 $mg.mL^{-1}$ of Irinotecan hydrochloride. As before, the loaded microspheres were placed in 200 mL PBS, while another 1 mL was placed in PBS with a reducing environment provided by DTT. The same method was followed as for the doxorubicin elutions in example 7. The results can be seen in Fig. 16. The results confirm that in the presence of a reducing environment the disulfide bonds will cleave leading to an increase in the rate of release of irinotecan.

**Example 12: Loading of Non-cationically charged Drugs**

There are many other drugs that may offer clinical benefits in the treatment of cancer by embolisation; however these drugs may not possess a positively charged group and therefore will not be loaded into these anionically charged

beads *via* a mechanism of ion exchange. A study was performed in order to load rapamycin into the BALC microsphere formulation using an alternative method.

The BALC microspheres were washed in dimethyl sulfoxide (DMSO) with an aliquot of 1 mL BALC microspheres washed 5 times with 1 mL DMSO. The DMSO causes the microspheres to swell, although the BALC within the formulation does not cleave. Once swollen a 60 $mg.mL^{-1}$ rapamycin solution, dissolved in DMSO, was added to a volume of 1 mL of beads. The drug was allowed to diffuse into the microsphere and after 10 minutes the excess solution was drained and the bead slurry was washed with deionised water. The BALC beads took on a white appearance as the drug precipitated within the bead. The beads loaded 10 $mg.mL^{-1}$ and drug was held within the bead within water but when placed in a PBS elution the drug was released from the microspheres. Figure 17 shows Rapamycin loaded BALC microspheres.

The BALC microspheres have also been loaded with other non-cationically charged drugs, such as dexamethasone and diclofenac using a similar process as that described for rapamycin. Fig. 18 shows Diclofenac loading of BALC microspheres.

**Example 13: Synthesis of BALC microparticles using comonomers other than PVA macromer**

BALC has also been used create copolymer microparticles with the use of alternative monomers. The microparticles were made using analogous methods to that described for the PVA macromer + BALC formulations, with the PVA being substituted in one study for 2-hydroxyethylmethacrylate (HEMA) (21 g HEMA + 2.5g BALC) and in another study using acrylic acid (AA) (21g AA + 2.5g BALC). In both examples, particulate or bead-like products could be isolated.

**Example 14: Loading and elution of doxorubicin from a BALC-HEMA formulation**

A sample of BALC-HEMA formulation was placed in a low concentration doxorubicin solution to investigate whether the microparticles would be able to load positively charged drug. 1 mL of BALC-HEMA loaded 8.8 $mg.mL^{-1}$ doxorubicin. The loaded microparticles where then used to study the release profile of the microparticles. The same method used for the BALC-PVA formulations in example 7 was applied. The results can be seen in Fig. 19.

The release profile again shows an increase in release in a reducing environment as expected. However the release was 2.6 times greater than the non-reducing environment. Images of the microparticles before elution, (Fig 20A) during elution in a normal environment (Fig. 20B) and in a reducing environment during elution (Fig. 20C) were taken. Figs. 20B and 20C were taken at 6 hours.

Fig. 20 clearly shows that the particles remain completely intact in the normoxic environment; however, the drug loaded particles have completely degraded in the reducing environment releasing all of the drug payload. This demonstrates that BALC copolymer formulations with lower molecular weight components can degrade more rapidly in a reducing environment.

**Example 15: Loading and elution of doxorubicin from a BALC-AA formulation**

The same experiment as per example 14 was carried out on the BALC-acrylic acid formulation. 1 mL microparticles loaded all of the doxorubicin 11.6 $mg.mL^{-1}$ added. The elution profile can be seen in Fig 21.

Within the first 6 hours only a slight increase in release was noted within a reducing environment, and it appears that this formulation has a very slow release. This could be due to the extra drug binding sites added by the acrylic acid and this is shown by the beads in a normal reducing environment throughout. However, at the 24 hour point of the release profile, there is again an exponential increase in drug release within the reducing environment. Visual observations showed that there were clear differences again in the appearances of the microparticles in different environments. Images of the beads before elution, (Fig 22A) during elution in a normal environment (Fig. 22B) and in a reducing environment during elution (Fig. 22C) were taken. Figs. 22B and 22C were taken at 45 hours.

Again, the beads in a non-reducing environment remain completely intact, but the microparticles in a reducing environment have degraded again leading to an increase in release. This again demonstrates that microparticles with copolymers with BALC can degrade in a reducing environment releasing the entire payload.

**Example 16: Biocompatibility of Non-drug loaded BALC microparticles with human liver cells**

An experiment was performed to investigate whether the microparticles would cause cell death by leaching of materials out of the microparticle and therefore showing they are not compatible within the body. The HEP G2 cell line was cultured and seeded into a 24 well plate at 20,000 cells/well with 1 mL cell MEM media (n=6). Once seeded, 10 of the 45% BALC microspheres were added to each well and left to incubate over a period of 24-72 hours. The experiment showed that the cells survived and replicated during the incubation period in the presence of the non-drug loaded

microspheres. This shows that the microspheres are biocompatible and have no cytotoxic effect.

## Claims

1. Embolic microparticles for use in a method of treatment by embolisation wherein in the treatment drug is released in a hypoxic environment, the microparticles comprising a gel body, wherein the gel body comprises a synthetic polymer and a drug, wherein the microparticles have an average diameter in the range 40 to 1500 μm, wherein the polymer is cross-linked by groups comprising disulphide linkages and is in the form of a hydrogel, wherein each cross linking group comprises two cationically or anionically charged groups at physiological pH, symmetrically arranged either side of the disulfide linkage, wherein the two cationically or anionically charged groups are electrostatically associated with the drug.

2. Embolic microparticles for use according to claim 1, wherein the polymer is cross-linked by a cross-linker derived from a cross-linking agent having the formula

$$A\text{-}(CH_2)n\text{-}S\text{-}S\text{-}(CH_2)n'\text{-}A'$$

   wherein A and A' are independently selected from groups which can form a covalent bond with the polymer; n and n' are integers ranging from 1 to 10; and wherein the $-(CH_2)n-$ and $-(CH_2)n'$ groups are optionally substituted with groups which are capable of electrostatically associating with an anionic or cationic drug.

3. Embolic microparticles for use according to claims 1 or 2, wherein in the method of treatment by embolisation, the microparticles are exposed to a hypoxic environment, in which the disulfide linkages are cleaved, facilitating release of the drug.

4. Embolic microparticles for use according to any preceding claims , wherein the method of treatment by embolisation is treatment of a tumour.

5. Embolic microparticles for use according to any preceding claim wherein the drug is selected from the group consisting of anti-tumour drugs, anti-angiogenesis drugs, anti-fungal drugs, antiviral drugs, anti-inflammatory drugs, anti-bacterial drugs, anti-histamine drugs, antineoplastic drugs, enzymes and anti-allergenic drugs, and is preferably, an anthracycline or camptothecin compound.

6. Embolic microparticles for use according to any preceding claim, wherein the microparticles are microspheres.

7. Embolic microparticles for use according to any preceding claim, wherein the polymer is an acrylic-based polymer.

8. Embolic microparticles for use according to any one of claims 1 to 6, wherein the polymer is a vinyl alcohol polymer.

9. Embolic microparticles for use according to any preceding claim which have an average diameter in the range 40-300μm.

10. Embolic microparticles for use according to any preceding claim, wherein the polymer is anionic, and the drug is cationic, and electrostatically associated with the polymer.

11. Embolic microparticles for use according to claim 10, wherein the groups which cross-link the polymer contribute to the anionic charge on the polymer.

12. Embolic microparticles for use according to any preceding claim wherein the polymer is formed from a PVA macromer.

13. Embolic microparticles for use according to any preceding claim wherein the microparticles are biodegradable.

14. A pharmaceutical composition comprising embolic microparticles according to any preceding claim.

15. A composition according to claim 14 further comprising a pharmaceutically acceptable liquid vehicle, preferably comprising physiological saline and/or a contrast agent visible by imaging devices, for instance x-ray.

**16.** Use of embolic microparticles as defined in any of the preceding claims in the manufacture of an embolic composition for use in a method of treatment by embolisation wherein the drug is released from the microparticle.

**17.** Use according to claim 16 wherein the method of treatment is of a tumour, preferably wherein the drug is released from the microparticle in a hypoxic environment.

**Patentansprüche**

**1.** Embolische Mikropartikel zur Verwendung in einem Behandlungsverfahren durch Embolisation, wobei bei der Behandlung Wirkstoff in einer hypoxischen Umgebung freigesetzt wird, wobei die Mikropartikel einen Gelkörper umfassen, wobei der Gelkörper ein synthetisches Polymer und einen Wirkstoff umfasst, wobei die Mikropartikel einen durchschnittlichen Durchmesser im Bereich von 40 bis 1500 μm aufweisen, wobei das Polymer durch Gruppen, die Disulfidbindungen umfassen, vernetzt ist und in Form eines Hydrogels vorliegt, wobei jede Vernetzungsgruppe zwei bei physiologischem pH-Wert kationisch oder anionisch geladene Gruppen umfasst, die symmetrisch auf beiden Seiten der Disulfidbindung angeordnet sind, wobei die beiden kationisch oder anionisch geladenen Gruppen elektrostatisch mit dem Wirkstoff assoziiert sind.

**2.** Embolische Mikropartikel zur Verwendung nach Anspruch 1, wobei das Polymer durch einen Vernetzer vernetzt ist, der von einem Vernetzungsmittel mit der folgenden Formel abgeleitet ist:

$$A\text{-}(CH_2)n\text{-}S\text{-}S\text{-}(CH_2)n'\text{-}A',$$

wobei A und A' unabhängig aus Gruppen ausgewählt sind, die eine kovalente Bindung mit dem Polymer bilden können;
n und n' ganze Zahlen im Bereich von 1 bis 10 sind; und wobei
die -($CH_2$)n- und -($CH_2$)n'-Gruppen wahlweise mit Gruppen substituiert sind, die elektrostatisch mit einem anionischen oder kationischen Wirkstoff assoziieren können.

**3.** Embolische Mikropartiel zur Verwendung nach Anspruch 1 oder 2, wobei bei dem Behandlungsverfahren durch Embolisation die Mikropartikel einer hypoxischen Umgebung ausgesetzt werden, in der die Disulfidbindungen gespalten werden, wodurch die Freisetzung des Wirkstoffs vereinfacht wird.

**4.** Embolische Mikropartikel zur Verwendung nach einem der vorhergehenden Ansprüche, wobei das Behandlungsverfahren durch Embolisation eine Behandlung eines Tumors ist.

**5.** Embolische Mikropartikel zur Verwendung nach einem der vorhergehenden Ansprüche, wobei der Wirkstoff aus der Gruppe bestehend aus Antitumor-Wirkstoffen, Antianiogenese- Wirkstoffen, antimykotischen Wirkstoffen, antiviralen Wirkstoffen, entzündungshemmenden Wirkstoffen, antibakteriellen Wirkstoffen, Antihistaminika, Antineoplastika, Enzymen und antiallergenen Wirkstoffen ausgewählt ist und vorzugsweise eine Anthracyclin- oder Camptothecin-Verbindung ist.

**6.** Embolische Mikropartikel zur Verwendung nach einem der vorhergehenden Ansprüche, wobei die Mikropartikel Mikrokugeln sind.

**7.** Embolische Mikropartikel zur Verwendung nach einem der vorhergehenden Ansprüche, wobei das Polymer ein Polymer auf Acrylbasis ist.

**8.** Embolische Mikropartikel zur Verwendung nach einem der Ansprüche 1 bis 6, wobei das Polymer ein Vinylalkoholpolymer ist.

**9.** Embolische Mikropartikel zur Verwendung nach einem der vorhergehenden Ansprüche, die einen durchschnittlichen Durchmesser im Bereich von 40-300 μm aufweisen.

**10.** Embolische Mikropartikel zur Verwendung nach einem der vorhergehenden Ansprüche, wobei das Polymer anionisch ist und der Wirkstoff kationisch und elektrostatisch mit dem Polymer assoziiert ist.

**11.** Embolische Mikropartikel zur Verwendung nach Anspruch 10, wobei die Gruppen, die das Polymer vernetzen, zur

anionischen Ladung auf dem Polymer beitragen.

**12.** Embolische Mikropartikel zur Verwendung nach einem der vorhergehenden Ansprüche, wobei das Polymer aus einem PVA-Makromer gebildet ist.

**13.** Embolische Mikropartikel zur Verwendung nach einem der vorhergehenden Ansprüche, wobei die Mikropartikel biologisch abbaubar sind.

**14.** Pharmazeutische Zusammensetzung, umfassend embolische Mikropartikel nach einem der vorhergehenden Ansprüche.

**15.** Zusammensetzung nach Anspruch 14, ferner umfassend ein pharmazeutisch annehmbares flüssiges Vehikel, vorzugsweise umfassend physiologische Kochsalzlösung und/oder durch bildgebende Vorrichtungen, beispielsweise Röntgenstrahlen, sichtbares Kontrastmittel.

**16.** Verwendung von embolischen Mikropartikeln nach einem der vorhergehenden Ansprüche bei der Herstellung einer embolischen Zusammensetzung zur Verwendung bei einem Behandlungsverfahren durch Embolisation, wobei der Wirkstoff aus dem Mikropartikel freigesetzt wird.

**17.** Verwendung nach Anspruch 16, wobei das Behandlungsverfahren das eines Tumors ist, wobei vorzugsweise der Wirkstoff in einer hypoxischen Umgebung aus dem Mikropartikel freigesetzt wird.

## Revendications

**1.** Microparticules emboliques pour une utilisation dans un procédé de traitement par embolisation, dans lesquelles le médicament du traitement est libéré dans un environnement hypoxique, les microparticules comprenant un corps de gel, dans lequel le corps de gel comprend un polymère synthétique et un médicament, dans lequel les microparticules ont un diamètre moyen dans la fourchette de 40 à 1500 μm, dans lequel le polymère est réticulé par des groupements comprenant des liaisons disulfures et il est sous la forme d'un hydrogel, dans lequel chaque groupement de réticulation comprend deux groupements chargés cationiquement ou anioniquement au pH physiologique, placés symétriquement de part et d'autre de la liaison disulfure, dans lequel les deux groupements chargés cationiquement ou anioniquement sont électrostatiquement associés au médicament.

**2.** Microparticules emboliques pour une utilisation selon la revendication 1, dans lesquelles le polymère est réticulé par un agent de réticulation dérivé d'un agent de réticulation ayant la formule

$$A\text{-}(CH_2)n\text{-}S\text{-}S\text{-}(CH_2)n'\text{-}A'$$

dans laquelle A et A' sont indépendamment choisis parmi les groupes qui peuvent former une liaison covalente avec le polymère ;
n et n' sont des entiers allant de 1 à 10 ; et dans lequel les groupements $-(CH_2)n-$ et $-(CH_2)n'$ sont éventuellement substitués par des groupements qui sont capables de s'associer électrostatiquement avec un médicament anionique ou cationique.

**3.** Microparticules emboliques pour une utilisation selon les revendications 1 ou 2, dans lesquelles dans le procédé de traitement par embolisation, les microparticules sont exposées à un environnement hypoxique, dans lequel les liaisons disulfure sont clivées, facilitant la libération du médicament.

**4.** Microparticules emboliques pour une utilisation selon l'une quelconque des revendications précédentes, dans lesquelles le procédé de traitement par embolisation est le traitement d'une tumeur.

**5.** Microparticules emboliques pour une utilisation selon une quelconque revendication précédente, dans lesquelles le médicament est choisi parmi le groupe composé des médicaments anti-tumoraux, des médicaments anti-angiogenèse, des médicaments antifongiques, des médicaments antiviraux, des médicaments anti-inflammatoires, des médicaments antibactérien, des médicaments antihistaminiques, des médicaments antinéoplasiques, des enzymes et des médicaments anti-allergéniques, et il est préférablement un composé anthracycline ou camptothécine.

6. Microparticules emboliques pour une utilisation selon une quelconque revendication précédente, dans lesquelles les microparticules sont des microsphères.

7. Microparticules emboliques pour une utilisation selon une quelconque revendication précédente, dans lesquelles le polymère est un polymère à base d'acrylique.

8. Microparticules emboliques pour une utilisation selon l'une quelconque des revendications 1 à 6, dans lesquelles le polymère est un polymère d'alcool vinylique.

9. Microparticules emboliques pour une utilisation selon une quelconque revendication précédente, qui ont un diamètre moyen dans la fourchette de 40 à 300 $\mu$m.

10. Microparticules emboliques pour une utilisation selon une quelconque revendication précédente, dans lesquelles le polymère est anionique, et le médicament est cationique, et électrostatiquement associé au polymère.

11. Microparticules emboliques pour une utilisation selon la revendication 10, dans lesquelles les groupements qui réticulent le polymère contribuent à la charge anionique du polymère.

12. Microparticules emboliques pour une utilisation selon une quelconque revendication précédente, dans lesquelles le polymère est formé d'un macromère PVA.

13. Microparticules emboliques pour une utilisation selon une quelconque revendication précédente, dans lesquelles les microparticules sont biodégradables.

14. Composition pharmaceutique comprenant les microparticules emboliques selon une quelconque revendication précédente.

15. Composition selon la revendication 14, comprenant également un support liquide pharmaceutiquement acceptable, de préférence comprenant la solution saline physiologique et/ou un agent de contraste visible par les dispositifs d'imagerie, par ex., les rayons X.

16. Utilisation des microparticules emboliques telle que définie dans l'une quelconque des revendications précédentes, dans la préparation d'une composition embolique pour une utilisation dans un procédé de traitement par embolisation dans lequel le médicament est libéré à partir d'une microparticules.

17. Utilisation selon la revendication 16, dans laquelle le procédé de traitement est le traitement d'une tumeur, de préférence, dans lequel le médicament est libéré à partir d'une microparticule dans un environnement hypoxique.

Figure 1

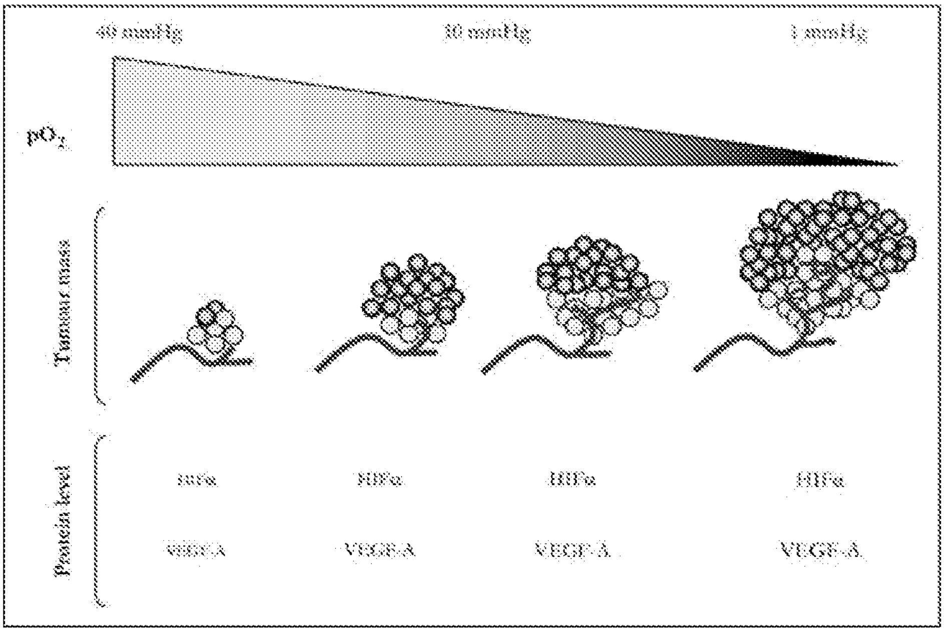
40 mmHg
10 mmHg
1 mmHg
$pO_2$
Protein level
HIFα
HIFα
HIFα
VEGF-A
VEGF-A
VEGF-A

## Figure 2A

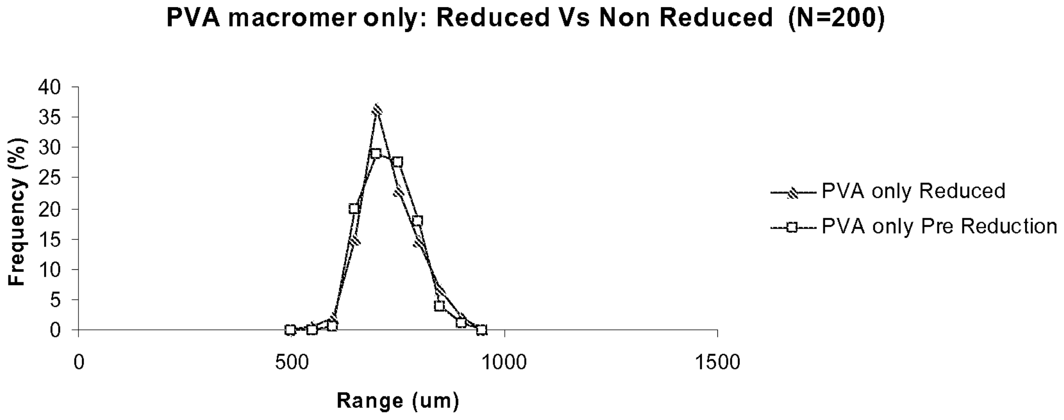


## Figure 2B

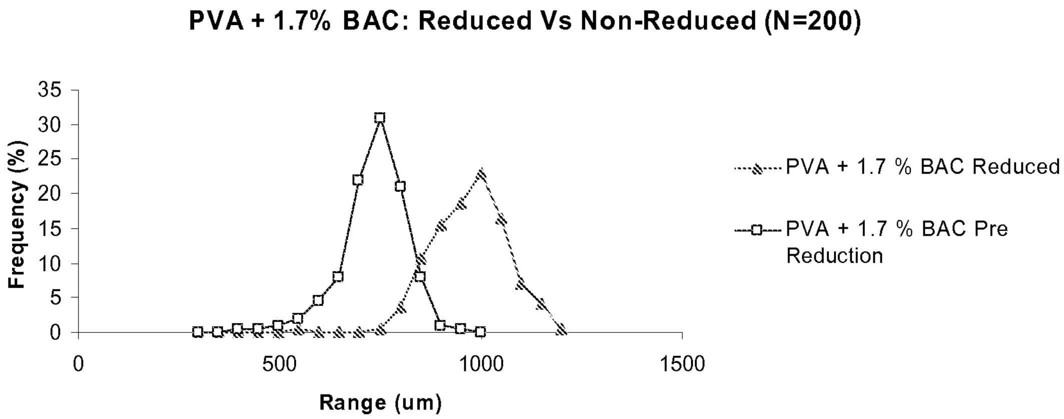


## Figure 3

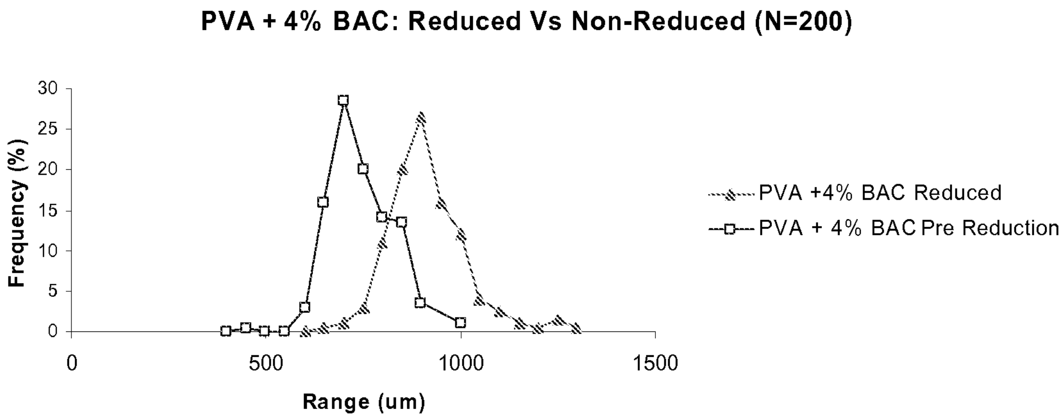

## Figure 4

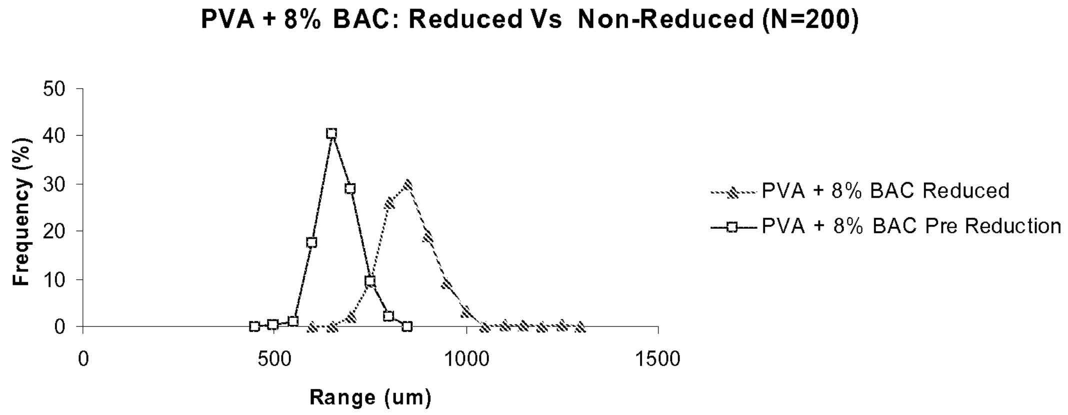


## Figure 5

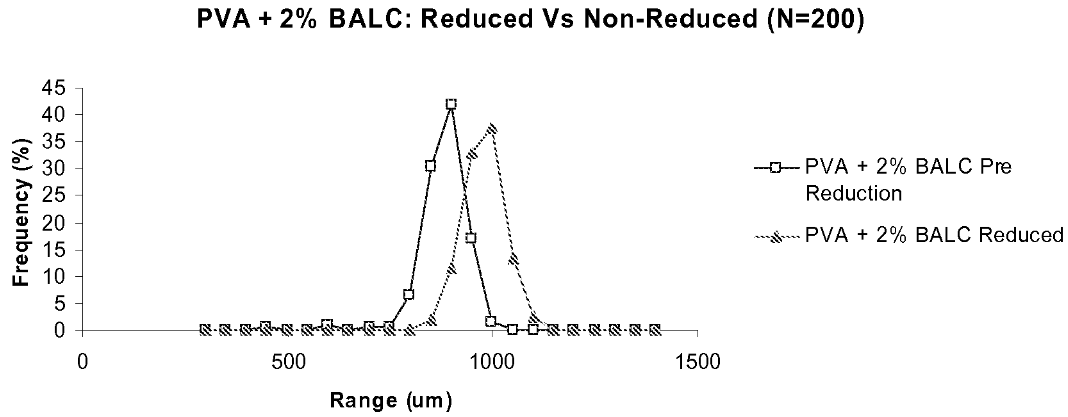


## Figure 6

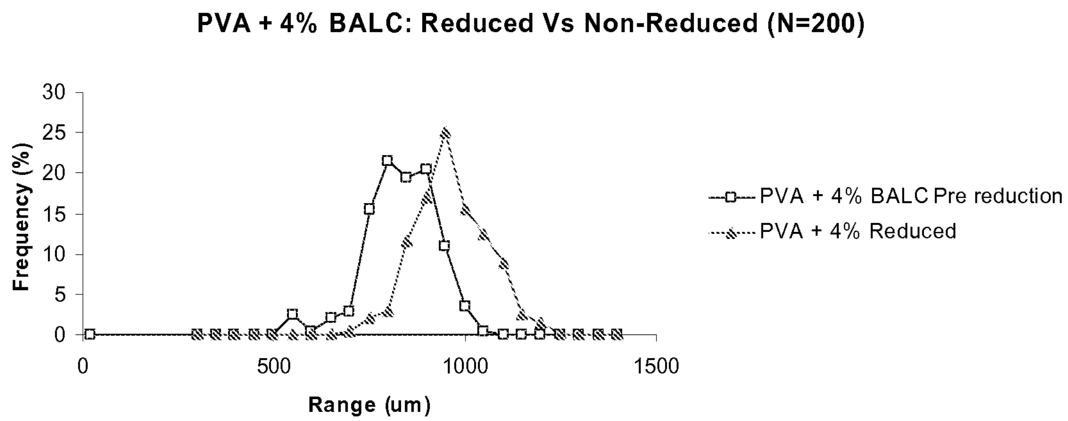

## Figure 7

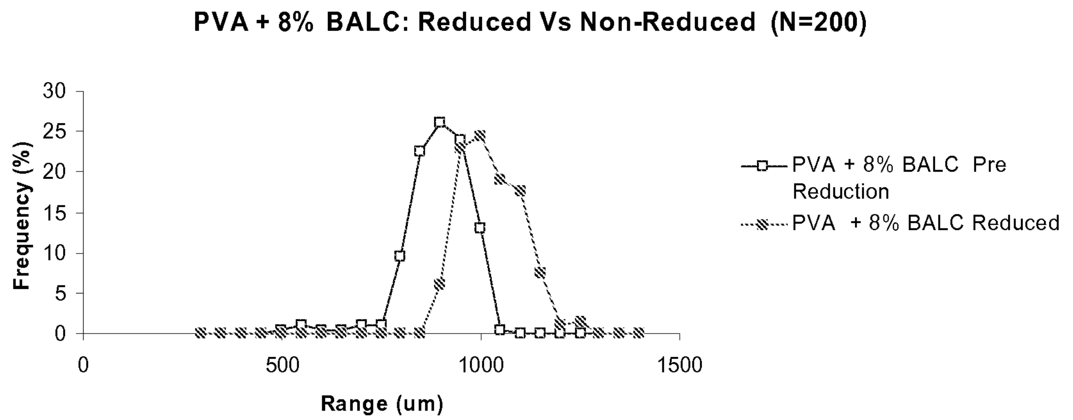


## Figure 8

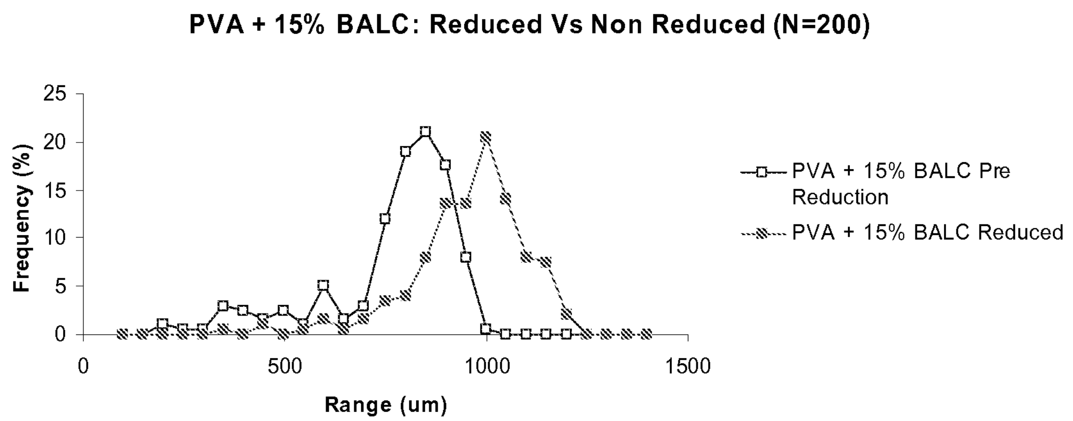

Figure 9

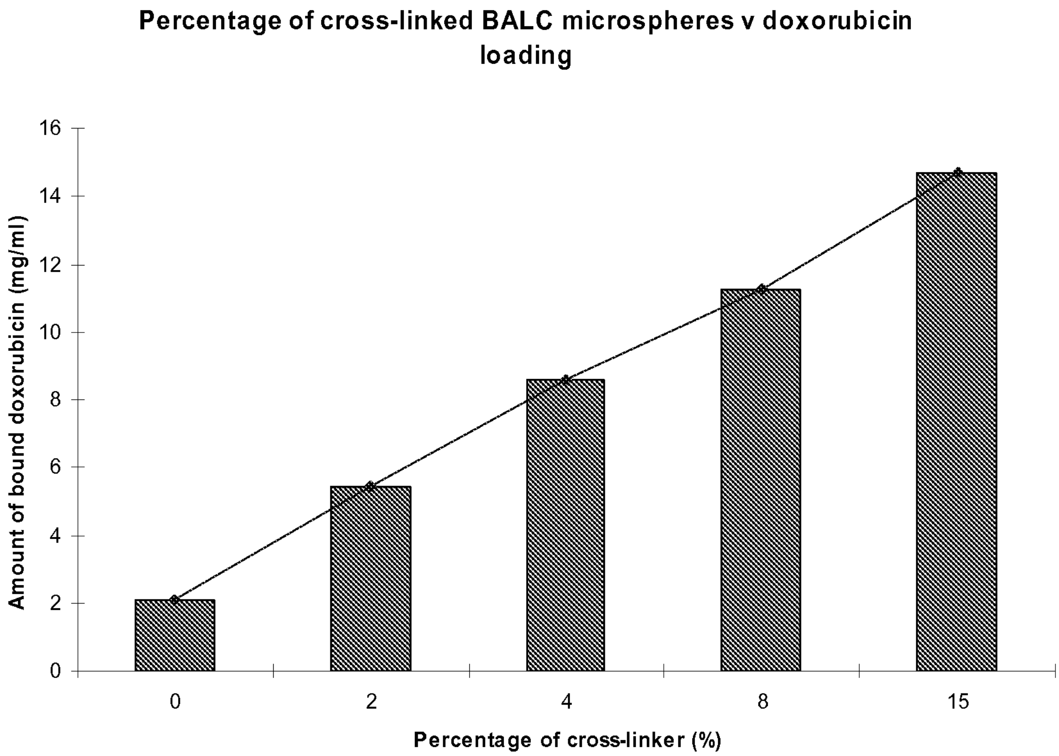


**Figure 10**: Picture of loaded beads after washing of doxorubicin. Left to right: 0%, 2%, 4%, 8% and 15% BALC microspheres

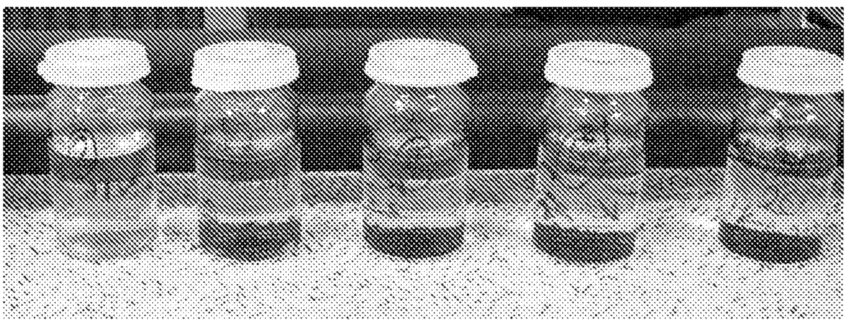

**Figure 11**

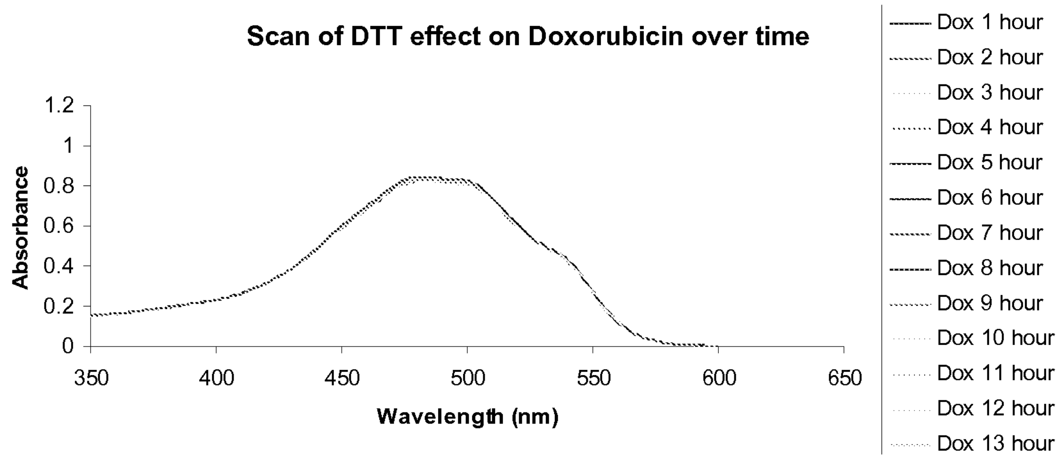


**Figure 12A**: Elution profile of doxorubicin from 31% BALC microspheres.

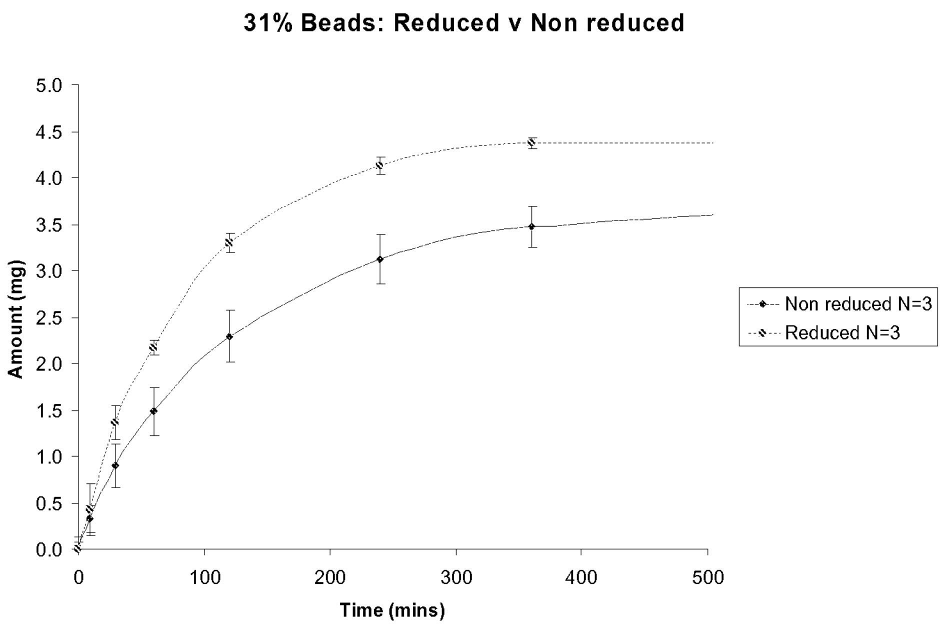

**Figure 12B**: Elution profile percentage release: Reduced BALC Vs Non-Reduced BALC

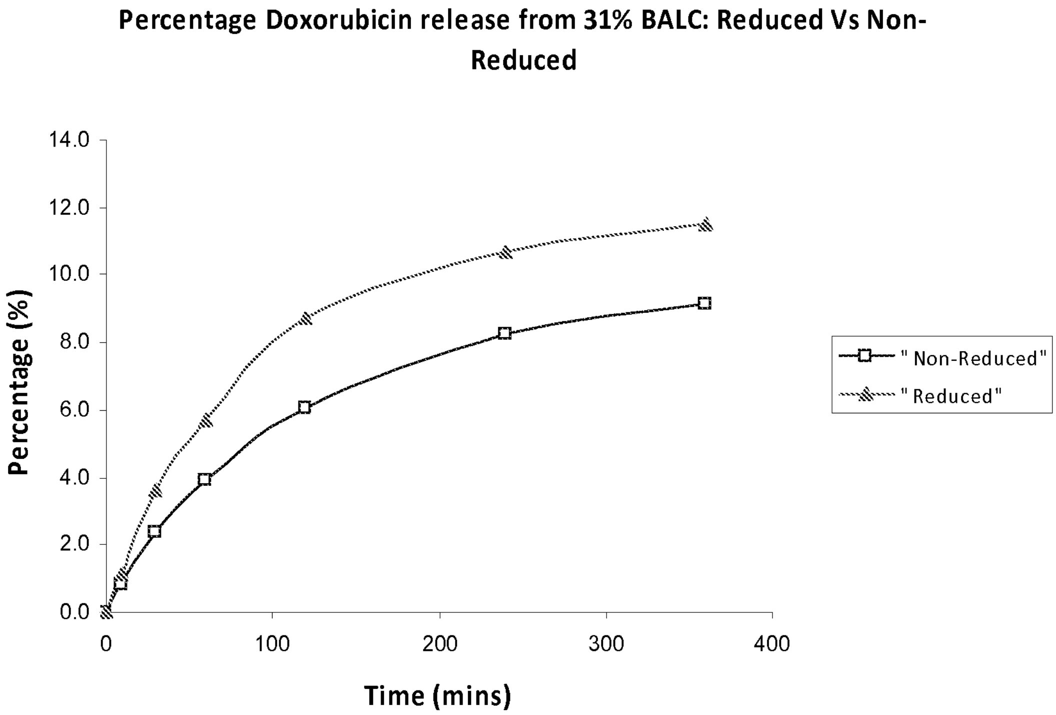


**Figure 13**

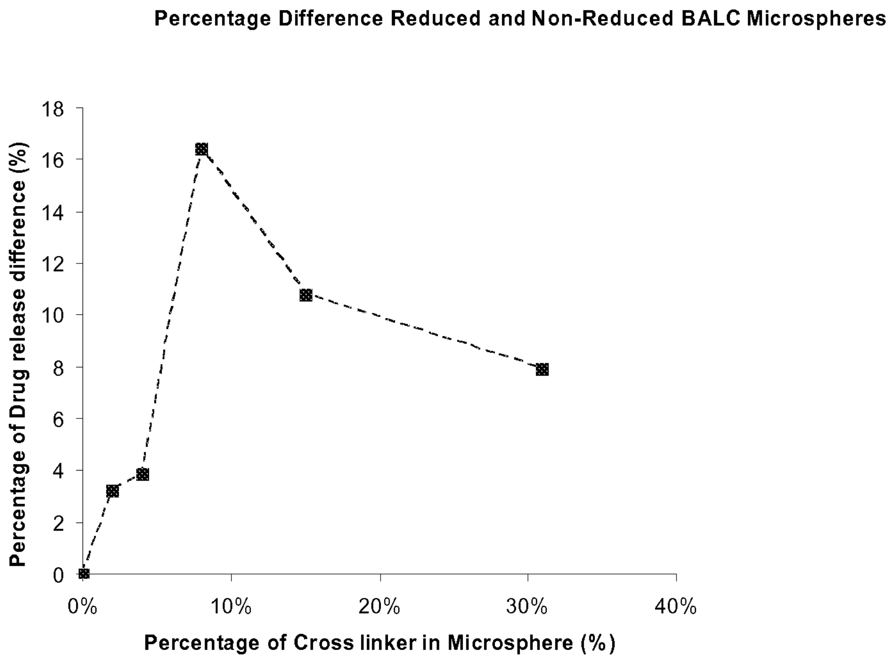

**Figure 14A**: Light microscope image of 100% BALC microparticles:

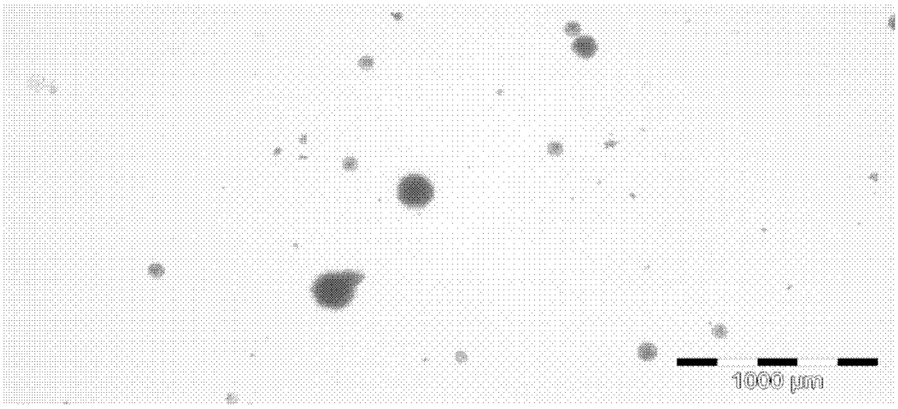


**Figure 14B**: Picture of 100% BALC microparticles settling in water

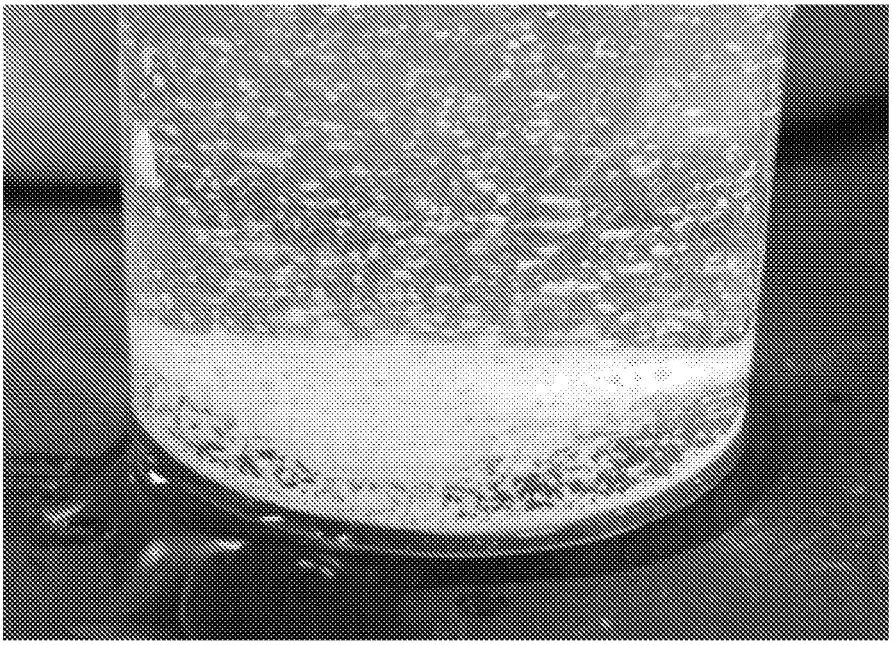

**Figure 15**: Size of the 45% BALC microspheres before and after reduction

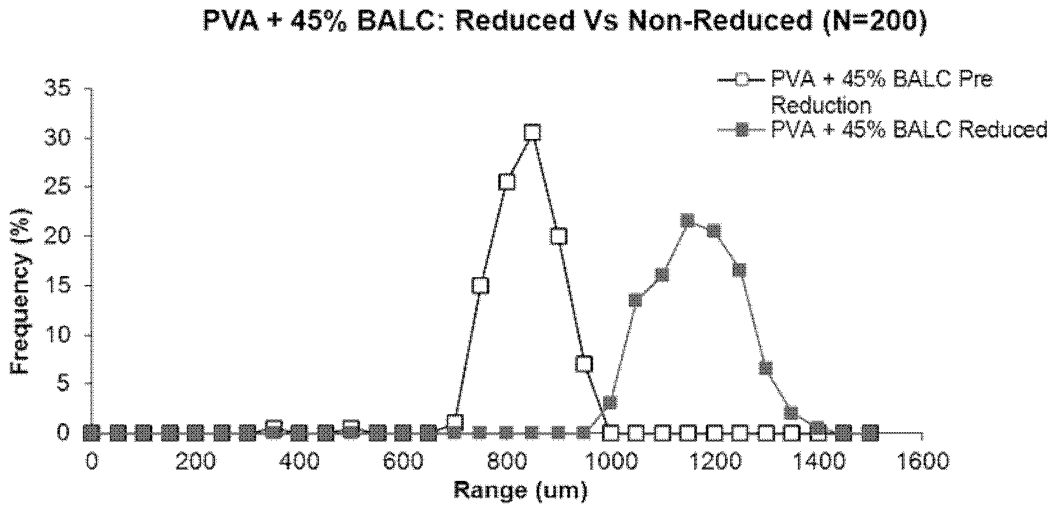


**Figure 16**: Elution profile of Irinotecan from 31% BALC microspheres (N=3)

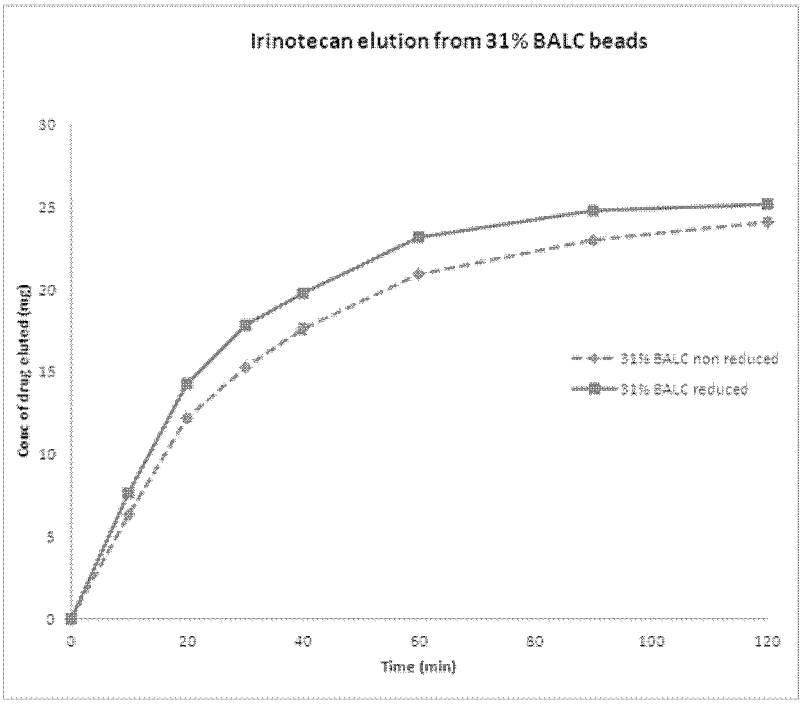

**Figure 17**: Rapamycin loaded BALC microspheres

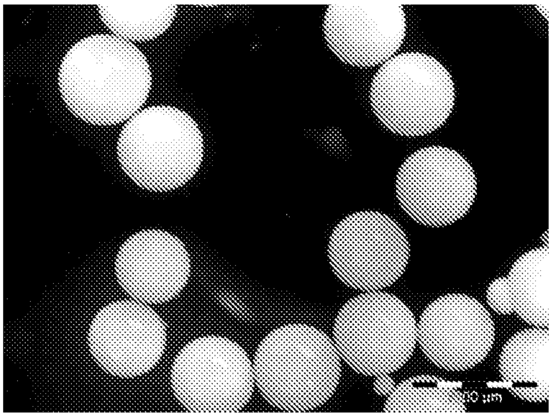

**Figure 18**: Diclofenac loaded BALC microspheres

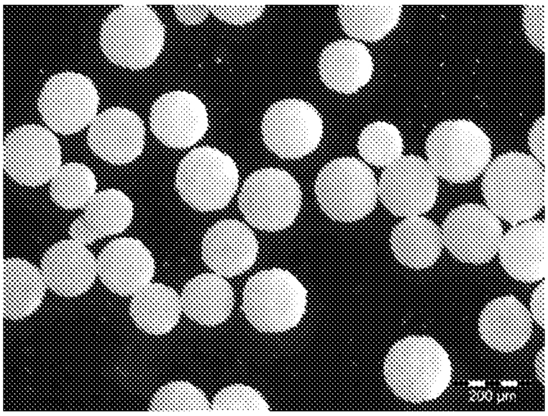


**Figure 19**: BALC-HEMA microparticle elution of doxorubicin

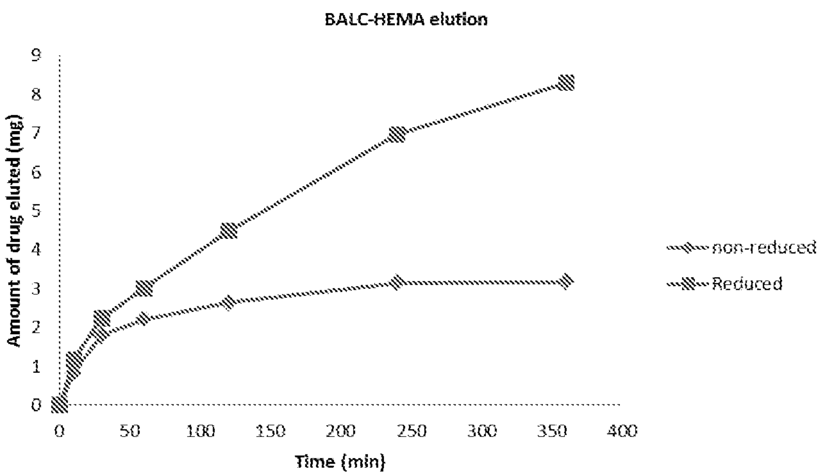

**Figure 20A**: BALC-HEMA microparticles loaded with drug prior to elution.

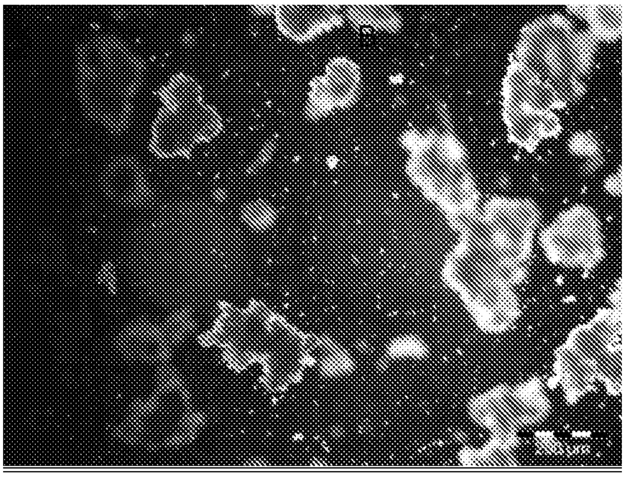

**Figure 20B**: BALC-HEMA microparticles during elution in a normal environment

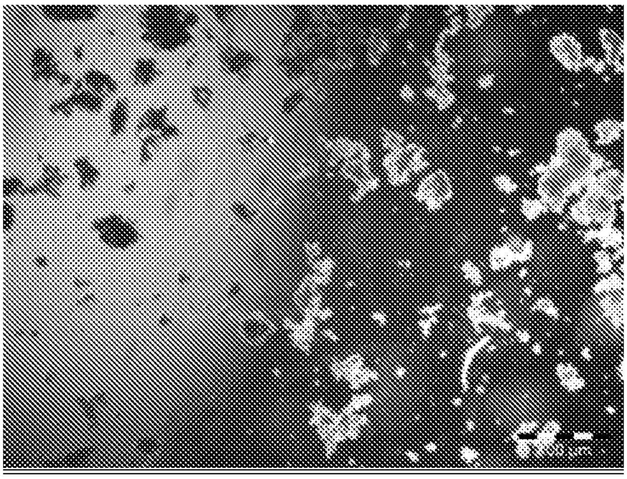

**Figure 20C**: BALC-HEMA microparticles during elution in a reducing environment

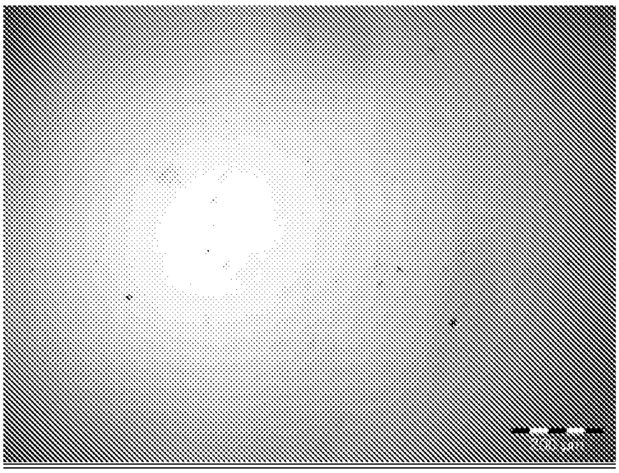

**Figure 21**: BALC-AA elution of doxorubicin

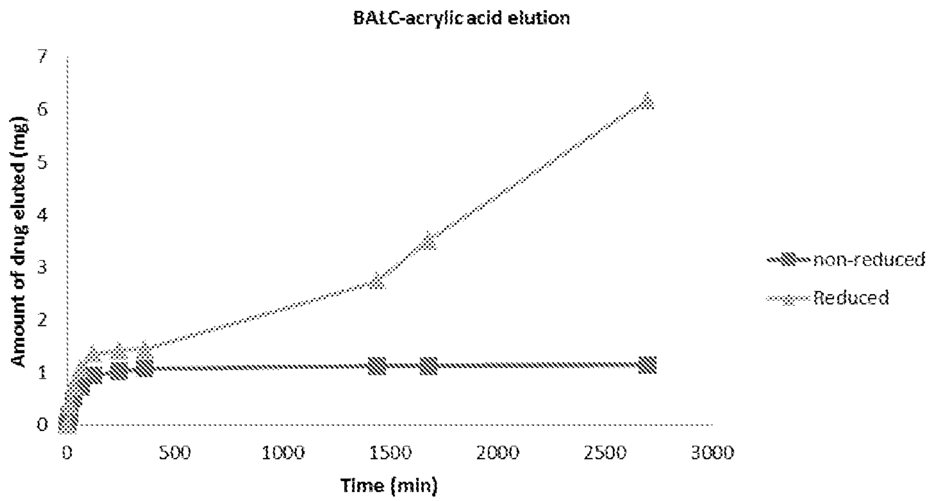

**Figure 22A**: BALC-acrylic acid microparticles loaded with drug prior to elution

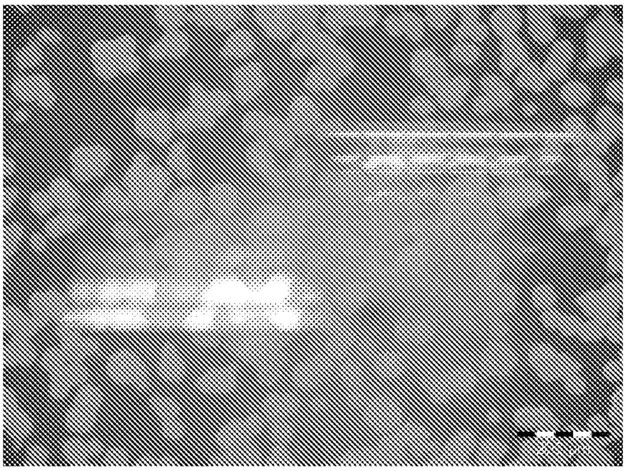

**Figure 22B**: BALC-acrylic acid microparticles during elution in a normal environment

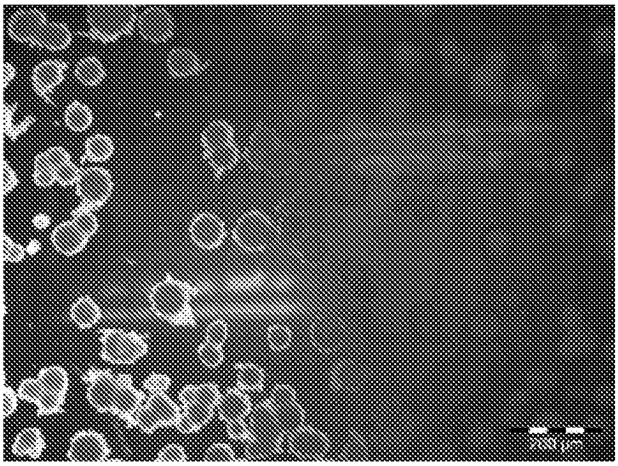

**Figure 22C**: BALC-acrylic acid microparticles during elution in a reducing environment

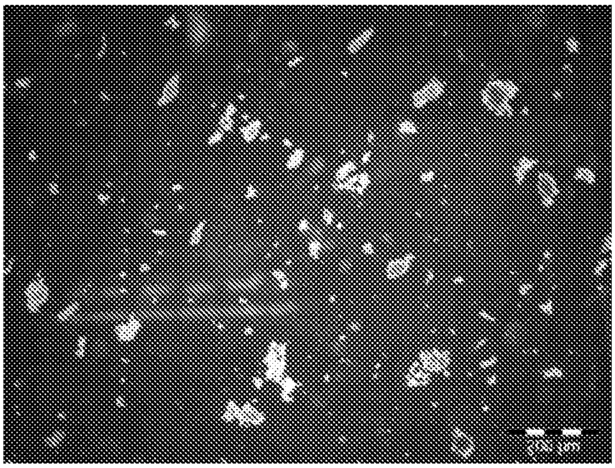

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20070231400 A, Quirk, S.
- EP 1007101 A2
- WO 04071495 A
- WO 0168720 A1
- US 4978713 A
- US 5508317 A
- US 5583163 A
- WO 2004071495 A
- WO 9207885 A
- WO 9416748 A
- WO 9416749 A
- WO 9520407 A
- US 4224427 A
- WO 2006027567 A
- WO 2003022807 A


### Non-patent literature cited in the description


- **MENG, F. et al.** Reduction-sensitive polymers and bioconjugates for biomedical applications. *Biomaterials,* 2009, vol. 30, 2180-2198
- **PATTERSON L.H.** AQ4 → AQ4N conversion under hypoxia. *Cancer Metastasis Rev.,* 1993, vol. 12, 119-34
- **OUPICKY, D.** Design and development strategies of polymer materials for drug and gene delivery applications. *Advanced Drug Delivery Reviews,* 2008, vol. 60, 957
- **MAK W.C. et al.** Protein particles formed by protein activation and spontaneous self-assembly. *Advanced Functional Materials,* 2010, vol. 20, 4139-4144
- **ZELIKIN, A.N. et al.** Disulfide cross-linked polymer capsules: en route to biodeconstructible systems. *Biomacromolecules,* 2006, vol. 7, 27-30
- **GREIMEL A. et al.** Oral peptide delivery: in vitro evaluation of thiolated alginate/poly(acrylic acid) microparticles. *J. Pharm. Pharmacol.,* 2007, vol. 59, 1191-8
- **BERNKOP-SCHNURCH A. et al.** Preparation and in vitro characterization of poly(acrylic acid)-cysteine microparticles. *J. Controlled Release,* 2003, vol. 18, 29-38
- **YAN, Y. et al.** Uptake and Intracellular fate of disulfide-bonded polymer hydrogel capsules for doxorubicin delivery to colorectal cancer cells. *ACS Nano,* 2010, vol. 4, 2928-36
- **LIANG, B. et al.** Correlation of hypoxia-inducible factor 1 alpha with angiogenesis in liver tumors after transcatheter arterial embolisation in an animal model. *CVIR,* 2010, vol. 33, 806-812
- New poly(amido amine)s containing disulfide linkages in their main chain. *Journal of polymer science Part A: polymer chemistry,* 2005, vol. 43 (7), 1404-1416
- Redox-Cleavable star cationic PDMAEMA by arm-first approach of ATRP as a nonviral vector for gene delivery. *Biomaterials,* 2010, vol. 3, 559-569